# EUROPÄISCHE PATENTSCHRIFT

(11) **EP 3 897 565 B1**
(45) Veröffentlichungstag und Bekanntmachung des Hinweises auf die Patenterteilung: **12.02.2025**
(21) Anmeldenummer: 19801536.4
(22) Anmeldetag: 07.11.2019
(51) Int. Cl.: A61K 8/898, A61K 8/81, A61K 8/36, A61K 8/365, A61K 8/46, A61K 8/55, A61K 8/23, A61K 8/20, A61Q 5/10, A61Q 5/06

(54) **SCHNELLES FÄRBESYSTEM AMODIMETHICON + POLYMER**
QUICK COLOR SYSTEM AMODIMETHICONE + POLYMER
SYSTÈME DE COULEUR RAPIDE AMODIMÉTHICONE + POLYMÈRE

(30) Priorität: 18.12.2018 DE 102018222024
(43) Veröffentlichungstag der Anmeldung: 27.10.2021
(73) Patentinhaber: Henkel AG & Co. KGaA, 40589 Düsseldorf (DE)
(72) Erfinder: NOWOTTNY, Marc, 41069 Mönchengladbach (DE); LECHNER, Torsten, 40764 Langenfeld (DE); BAUMANN, Sofie, 42859 Remscheid (DE)
(86) Internationale Anmeldenummer: PCT/EP2019/080583
(87) Internationale Veröffentlichungsnummer: WO 2020/126204

(56) Entgegenhaltungen:
- DE-A1- 102004 013 795
- DE-A1- 102013 226 102
- DE-A1- 102014 218 006
- JP-A- 2008 019 204

## Beschreibung

Gegenstand der vorliegenden Anmeldung ist ein Verfahren zum Färben von keratinischem Material, insbesondere menschlichen Haaren, welches die Anwendung von mindestens zwei verschiedenen Mitteln (a) und (b) umfasst. Das Mittel (a) enthält mindestens ein aminofunktionalisiertes Silikonpolymer (a1) und mindestens eine farbgebende Verbindung (a2) aus der Gruppe der anorganischen und/oder organischen Pigmente,. Bei dem Mittel (b) handelt es sich um ein saures, wasserhaltiges Nachbehandlungsmittel mit einem pH-Wert von 1,5 bis 5,5. Weiterhin ist in mindestens einem der Mittel (a) und/oder (b) ein filmbildendes Polymer enthalten. Zunächst wird das Mittel (a) angewendet, danach wird das Mittel (b) angewendet, wobei der Zeitraum zwischen der Anwendung der Mittel (a) und (b) bei maximal 24 Stunden liegt.

Die Veränderung von Form und Farbe von keratinischem Material, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

DE 102014218006 A1 beschreibt eine Verpackungseinheit zur Färbung von Keratinfasern, welche die beiden Mittel (M1) und (M2) umfasst. Das Mittel (M1) enthält mindestens eine farbgebende Verbindung aus der Gruppe der Oxidationsfarbstoffvorprodukte und der direktziehenden Farbstoffe sowie ein Aminosilikon. Das Mittel (M2) ist eine Oxidationsmittelzubereitung.

DE 102004013795 A1 beschreibt kosmetische Mittel enthaltend mindestens ein Aminosilikon einer Formel (S_{H}).

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbeverfahren.

Es war die Aufgabe der vorliegenden Erfindung, ein Färbesystem bereitzustellen, das mit der oxidativen Färbung vergleichbare Echtheitseigenschaften besitzt. Insbesondere die Waschechtheiten sollten herausragend sein, hierbei sollte jedoch auf den Einsatz der sonst zu diesem Zweck üblicherweise eingesetzten Oxidationsfarbstoffvorprodukte verzichtet werden. Es wurde nach einer Technologie gesucht, die es ermöglicht, die aus dem Stand der Technik bekannten farbgebenden Verbindungen (wie beispielsweise Pigmente) in extrem dauerhafter Weise auf den Haaren zu fixieren. Bei Anwendung der Mittel in einem Färbeverfahren sollten intensive Färbeergebnisse mit guten Echtheitseigenschaften erzielt werden. Insbesondere sollten bei Anwendung der entsprechenden Verfahren besonders waschechte Färbungen erhalten werden, die auch nach wiederholtem Kämmen oder Frisieren keine Abschwächung der Farbintensität erleiden.

Überraschenderweise hat sich nun herausgestellt, dass die vorgenannte Aufgabe hervorragend gelöst werden kann, wenn keratinische Materialien, insbesondere Haare, mit einem Verfahren gefärbt werden, bei welchem mindestens zwei Mittel (a) und (b) auf die keratinischen Materialien (Haare) appliziert werden. Hierbei enthält das Mittel (a) mindestens ein aminofunktionalisiertes Silikonpolymer (a1), mindestens eine farbgebende Verbindung (a2) aus der Gruppe der anorganischen und/oder organischen Pigmente und gegebenenfalls mindestens ein von (a1) verschiedenes filmbildendes Polymer (a3). Das Mittel (b) stellt ein saures Nachbehandlungsmittel mit einem pH-Wert von 1,5 bis 5,5 dar und enthält Wasser, mindestens eine Säure (b1) sowie gegebenenfalls mindestens ein filmbildendes Polymer (b2). Weiterhin besteht bei beiden Mitteln (a) und (b) die Maßgabe, dass mindestens eines der Mittel (a) und/oder (b) mindestens ein filmbildendes Polymer enthält. Zunächst wird das Mittel (a) angewendet, danach wird das Mittel (b) angewendet, und der Zeitraum zwischen der Anwendung der Mittel (a) und (b) liegt bei maximal 24 Stunden. Bei Einsatz der beiden Mittel (a) und (b) in einem Färbeverfahren konnten keratinische Fasern mit hoher Farbintensität gefärbt werden. Zudem war die Waschechtheit der mit (a) und (b) gefärbten Haare hervorragend.

Ein erster Gegenstand der vorliegenden Erfindung ist ein Verfahren zum Färben von keratinischem Material, insbesondere menschlichen Haaren, umfassend die folgenden Schritte:
- Anwendung eines Färbemittels (a) auf dem keratinischem Material, wobei das Mittel (a) enthält:
   (a1) mindestens ein aminofunktionalisiertes Silikonpolymer, und
   (a2) mindestens eine farbgebende Verbindung aus der Gruppe der anorganischen und/oder organischen Pigmente, und
   (a3) gegebenenfalls mindestens ein filmbildendes Polymer, das von (a1) verschieden ist,
- Anwendung eines Nachbehandlungsmittels (b) auf dem keratinischen Material, wobei das Mittel (b) einen pH-Wert von 1,5 bis 5,5 besitzt und enthält: Wasser und
   (b1) mindestens eine Säure, und
   (b2) gegebenenfalls mindestens ein filmbildendes Polymer,

mit der Maßgabe, dass mindestens eines der Mittel (a) und/oder (b) mindestens ein filmbildendes Polymer enthält,
dadurch gekennzeichnet, dass zunächst das Mittel (a) angewendet wird, danach das Mittel (b) angewendet wird, wobei der Zeitraum zwischen der Anwendung der Mittel (a) und (b) bei maximal 24 Stunden liegt.

Bei den zu dieser Erfindung führenden Arbeiten hat sich gezeigt, dass die sukzessive Anwendung von Färbemittel (a) und Nachbehandlungsmittel (b) zu gefärbtem keratinischen Material führt, welches sich durch gute Echtheitseigenschaften, insbesondere durch besonders gute Waschechtheiten auszeichnet.

Ohne auf diese Theorie beschränkt zu sein, wird vermutet, dass das im Färbemittel (a) enthaltene aminofunktionalisiertes Silikonpolymer (a1) mit dem Keratinmaterial adhäsive Bindungen eingeht, die das Aminosilikon (a1) zuächst auf dem Keratinmaterial fixieren. Die farbgebenden Verbindungen (a2) lagern sich hierbei in oder an das Aminosilikon (a1) ein und werden auf diese Weise ebenfalls an der Außenseite des Keratinmaterials immobilisiert.

Die adhäsiven Bindungen zwischen aminofunktionalisiertem Silikonpolymer (a1) und Keratin basieren vermutlich auf elektrostatischen Wechselwirkungen, die sich zwischen den positiv geladenen Aminogruppen des Silikon-Polymers (a1) und negativen Ladungen auf dem Keratinmaterial ausbilden.

Überraschenderweise konnte beobachtet werden, dass die farbgebenden Verbindungen (a2) sich bei gleichzeitiger Anwendung mit dem Aminosilikon (a1) an letztes anlagern oder mit diesem eine gemeinsame Schicht ausbilden. Diese gemeinsame Schichtbildung von (a1) und (a2) führt dazu, dass Färbungen mit guter Waschechheit erhalten werden können, auch ohne dass eine Diffusion der farbgebenden Verbindung in die Haarfaser hinein notwendig wäre. Durch Einsatz eines filmbildenden Polymers, dass entweder in das Färbemittel (a) und/oder in das Nachbehandlungsmittel (b) eingearbeitet wird, kann die Schicht aus (a1) und (a2) zusätzlich fixiert und die Waschechtheit auf diese Weise weiter verstärkt werden.

Des weiteren hat sich herausgestellt, dass bei der gemeinsamen Ablagerung von Aminosilikon (a1) und farbgebender Verbindung (a2) insbesondere dann sehr stabile Schichten ausgebildet werden konnten, wenn für die Anwendung die optimierten pH-Werte gewählt wurden. Besonders resistente Schichten konnten erzielt werden, wenn zunächst (a1) und (a2) bei neutralem bis basischem Milieu auf die Keratinmaterialien appliziert wurden, und der pH-Wert im Anschluss daran durch Anwendung eines sauren Nachbhenadlungsmittel abgesenkt wurde.

### keratinisches Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

### Mittel (a) und (b)

Im Rahmen des erfindungsgemäßen Verfahrens werden die Mittel (a) und (b) auf dem keratinischen Material, insbesondere den menschlichen Haaren, appliziert. Die beiden Mittel (a) und (b) sind voneinander verschieden.

Mit anderen Worten ist ein erster Gegenstand der vorliegenden Erfindung ein Verfahren zum Färben von keratinischem Material, insbesondere menschlichen Haaren, umfassend die folgenden Schritte:
- Anwendung eines Färbemittels (a) auf dem keratinischem Material, wobei das Mittel (a) enthält:
   (a1) mindestens ein aminofunktionalisiertes Silikonpolymer, und
   (a2) mindestens eine farbgebende Verbindung aus der Gruppe der anorganischen und/oder organischen Pigmente, und
   (a3) gegebenenfalls mindestens ein filmbildendes Polymer, das von (a1) verschieden ist,
- Anwendung eines Nachbehandlungsmittels (b) auf dem keratinischen Material, wobei das Mittel (b) einen pH-Wert von 1,5 bis 5,5 besitzt und enthält: Wasser und
   (b1) mindestens eine Säure, und
   (b2) gegebenenfalls mindestens ein filmbildendes Polymer,

mit der Maßgabe, dass
   - mindestens eines der Mittel (a) und/oder (b) mindestens ein filmbildendes Polymer enthält, und
   - die Mittel (a) und (b) voneinander verschieden sind,
dadurch gekennzeichnet, dass zunächst das Mittel (a) angewendet wird, danach das Mittel (b) angewendet wird, wobei der Zeitraum zwischen der Anwendung der Mittel (a) und (b) bei maximal 24 Stunden liegt.

### aminofunktionalisiertes Silikonpolymer (a1) im Mittel (a)

Als ersten erfindungswesentlichen Inhaltsstoff (a1) enthält das Mittel (a) mindestens ein aminofunktionalisiertes Silikonpolymer. Das aminofunktionalisiertes Silikonpolymer kann alternativ auch als Aminosilikon oder Amodimethicone bezeichnet werden.

Silikonpolymere sind im allgemeinen Makromoleküle mit einem Molekulargewicht von mindestens 500 g/mol, bevorzugt mindestens 1000 g/mol, weiter bevorzugt von mindestens 2500 g/mol, besonders bevorzugt von mindestens 5000 g/mol, welche sich wiederholende organische Einheiten umfassen.

Das maximale Molekulargewicht des Silikonpolymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des Silikonpolymers nicht mehr als 10⁷ g/mol, bevorzugt nicht mehr als 10⁶ g/mol und besonders bevorzugt nicht mehr als 10⁵ g/mol beträgt.

Die Silikonpolymere umfassen viele Si-O-Wiederholungseinheiten, wobei die Si-Atome organische Reste wie beispielsweise Alkylgruppen oder substituierte Alkylgruppen tragen können. Alternativ wird ein Silikonpolymer daher auch als Polydimethylsiloxan bezeichnet.

In Entsprechung des hohen Molekulargewichts der Silikonpolymere basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten, ganz besonders bevorzugt mehr als 500 Si-O-Wiederholungseinheiten.

Unter einem aminofunktionalisierten Silikonpolymer wird ein funktionalisiertes Polydimethylsiloxan verstanden, welches mindestens eine Struktureinheit mit einer Aminogruppe trägt. Bevorzugt trägt das aminofunktionalisierte Silikonpolymer mehrere Struktureinheiten mit jeweils mindestens einer Aminogruppe. Unter einer Aminogruppe wird eine primäre Aminogruppe, eine sekundäre Aminogruppe und eine tertiäre Aminogruppe verstanden. Alle diese Aminogruppen können im sauren Milieu protoniert werden und liegen dann in ihrer kationischen Form vor.

Prinzipiell konnten gute Effekte aminofunktionalisierten Silikonpolymeren (a1) erzielt werden, wenn diese mindestens eine primäre, mindestens eine sekundäre und/oder mindestens eine tertiäre Aminogruppe tragen. Färbungen mit der besten Waschechtheit wurden jedoch beobachtet, wenn ein aminofunktionalisiertes Silikonpolymer (a1) im Mittel (a) eingesetzt wurde, welches mindestens eine sekundäre Aminogruppe enthält.

In einer ganz besonders bevorzugten Ausführungsform ist ein erfindungsgemäßes Verfahren dadurch gekennzeichnet, dass das Mittel (a) mindestens ein aminofunktionalisiertes Silikonpolymer (a1) mit mindestens eine sekundären Aminogruppe enthält.

Die sekundäre Aminogruppe(n) kann bzw. können sich an verschiedenen Positionen des aminofunktionalisierten Silikonpolymers befinden. Ganz besonders gute Effekt wurden gefunden, wenn ein aminofunktionalisiertes Silikonpolymer (a1) eingesetzt wurde, dass mindestens eine, bevorzugt mehrere Struktureinheiten der Formel (Si-Amino) besitzt.

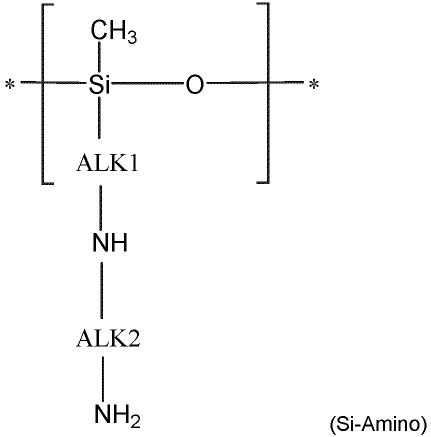

In den Struktureinheiten der Formel (Si-Amino) steht die Kürzel ALK1 und ALK2 unabhängig voneinander für eine lineare oder verzweigte, zweiwertige C₁-C₂₀-Alkylengruppe.

In einer weiteren ganz besonders bevorzugten Ausführungsform ist ein erfindungsgemäßes Verfahren dadurch gekennzeichnet, dass das Mittel (a) mindestens ein aminofunktionalisiertes Silikonpolymer (a1) enthält, das mindestens eine Struktureinheit der Formel (Si-Amino) umfasst, 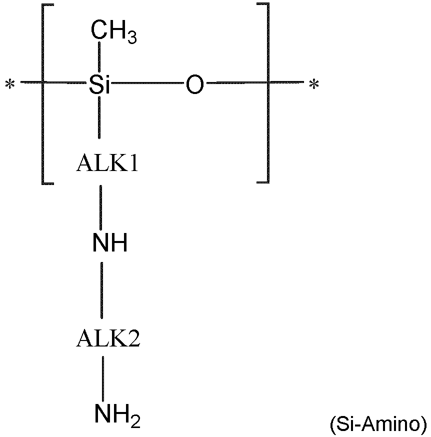 wobei
- ALK1 und ALK2: unabhängig voneinander für eine lineare oder verzweigte, zweiwertige C₁-C₂₀-Alkylengruppe stehen.

Die mit einem Stern (*) gekennzeichneten Position geben hierbei jeweils die Bindung zu weiteren Struktureinheiten des Silikonpolymers an. Beispielsweise kann das dem Stern benachbarte Silicium-Atom an ein weiteres Sauerstoffatom gebunden sein, und das dem Stern benachbarte Sauerstoffatom kann an ein weiteres Siliciumatom oder auch an eine C₁-C₆-Alkylgruppe gebunden sein.

Eine zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung ALK1 bzw. AK2 zwei Bindungen eingehen kann.

Im Fall von ALK1 erfolgt eine Bindung vom Silicium-Atom zur Gruppierung ALK1, und die zweite Bindung besteht zwischen ALK1 und der sekundären Aminogruppe.

Im Fall von ALK2 erfolgt eine Bindung von der sekundären Aminogruppe zur Gruppierung ALK2, und die zweite Bindung besteht zwischen ALK2 und der primären Aminogruppe.

Beispiele für eine lineare zweiwertige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweiwertige Alkylengruppen auch verzweigt sein. Beispiele für verzweigte, zweiwertige C₃-C₂₀-Alkylengruppen sind (-CH₂-CH(CH₃)-) und (-CH₂-CH(CH₃)-CH₂-).

In einer weiteren besonders bevorzugten Ausführungsform stellen die Struktureinheiten der Formel (Si-Amino) Wiederholungseinheiten im aminofunktionalisierten Silikonpolymer (a1) dar, so dass das Silikonpolymer mehrer Struktureinheiten der Formel (Si-Amino) umfasst.

Im Folgenden werden besonders gut geeignete aminofunktionalisierte Silikonpolymere (a1) mit mindestens einer sekundären Aminogruppe aufgelistet.

Färbungen mit den allerbesten Waschechtheiten konnten erhalten werden, wenn im erfindungsgemäßen Verfahren mindestens ein Mittel (a) auf dem keratinischen Material appliziert wurde, das mindestens ein aminofunktionalisiertes Silikonpolymer (a1) enthält, das Struktureinheiten der Formel (Si-I) und der Formel (Si-II) umfasst

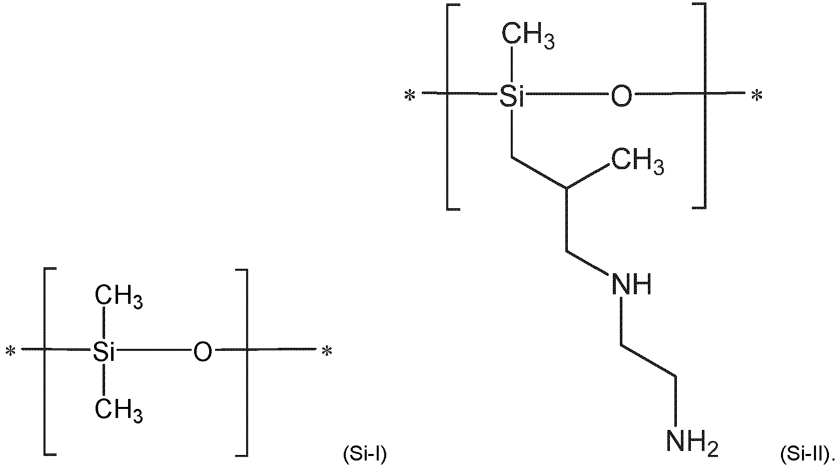

In einer weiteren explizit ganz besonders bevorzugten Ausführungsform ist ein erfindungsgemäßes Verfahren dadurch gekennzeichnet, dass das Mittel (a) mindestens ein aminofunktionalisiertes Silikonpolymer (a1) enthält, das Struktureinheiten der Formel (Si-I) und der Formel (Si-II) umfasst

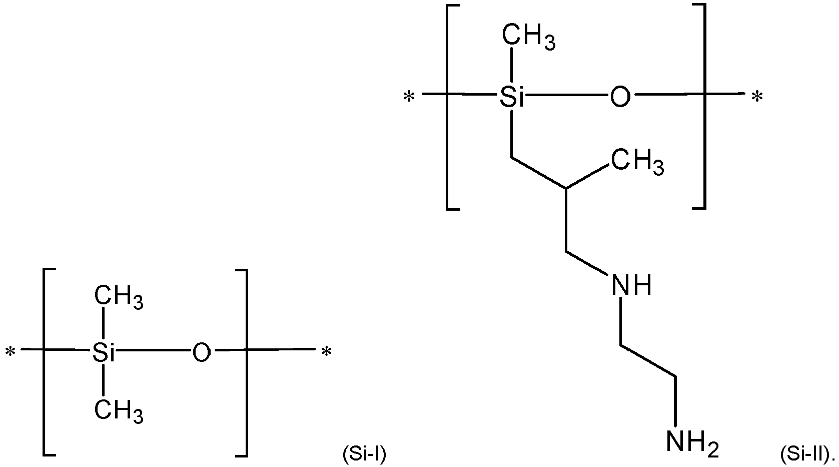

Ein entsprechendes aminofunkionalisiertes Silikonpolymer mit den Struktureinheiten (Si-I) und (Si-II) ist beispielweise das Handelsprodukt DC 2-8566 bzw. Dowsil 2-8566 Amino Fluid, das von der Firma Dow Chemical Company komerziell vertrieben wird und welches die Benennung "Siloxanes and Silicones, 3-[(2-Aminoethyl)amino]-2-methylpropyl Me, Di-Me-Siloxane" sowie die CAS-Nummer 106842-44-8 trägt.

Im Rahmen einer weiteren bevorzugten Ausführungsform ist ein erfindungsgemäßes Verfahren gekennzeichnet durch die Anwendung eines Mittels (a) auf dem keratinischem Material, wobei das Mittel (a) mindestens ein aminofunktionelles Silikonpolymer (a1) der Formel der Formel (Si-III) enthält, 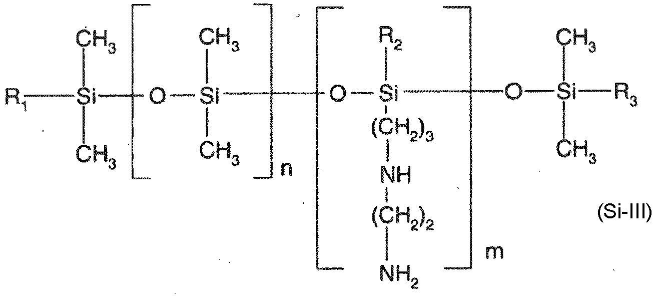 wobei
- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

Weitere erfindungsgemäß bevorzugte Verfahren sind gekennzeichnet durch die Anwendung eines Mittels (a) auf dem keratinischem Material, wobei das Mittel (a) mindestens aminofunktionelles Silikonpolymer (a1) der Formel der Formel (Si-IV) enthält, 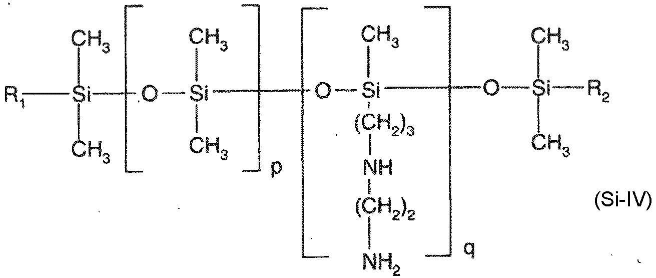 in der
- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

Die Silikone der Formeln (Si-III) und (Si-IV) unterscheiden sich durch die Gruppierung am Si-Atom, das die stickstoffhaltige Gruppe trägt: In Formel (Si-III) bedeutet R2 eine Hydroxygruppe oder eine C1-4-Alkoxygruppe, während der Rest in Formel (Si-IV) eine Methylgruppe ist. Die einzelnen Si-Gruppierungen, die mit den Indices m und n bzw. p und q gekennzeichnet sind, müssen nicht als Blöcke vorliegen, vielmehr können die einzelnen Einheiten auch statistisch verteilt vorliegen, d.h. in den Formeln (Si-III) und (Si-IV) ist nicht zwingend jedes R1-Si(CH₃)₂-Gruppe an eine -[O-Si(CH₃)₂]-Gruppierung gebunden.

Als besonders wirkungsvoll im Hinblick auf die gewünschten Effekte haben sich auch erfindungsgemäßen Verfahren erwiesen, in welchen ein Mittel (a) auf den Keratinfasern appliziert wird, welches mindestens ein aminofunktionelles Silikonpolymer (a1) der Formel der Formel (Si-V) enthält 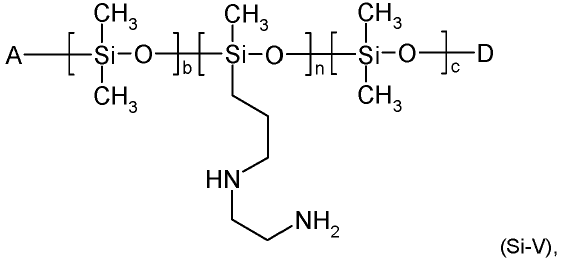 in der
- A: für eine Gruppe -OH, -O-Si(CH₃)₃,-O-Si(CH₃)₂OH ,-O-Si(CH₃)₂OCH₃ steht,
- D: für eine Gruppe -H, -Si(CH₃)₃,-Si(CH₃)₂OH, -Si(CH₃)₂OCH₃ steht,
- b, n und c: für ganze Zahlen zwischen 0 und 1000 stehen, mit den Maßgaben
- n > 0 und b + c > 0
- mindestens eine der Bedingungen A = -OH bzw. D = -H ist erfüllt.

In der vorstehend genannten Formel (Si-V) sind die einzelnen Siloxaneinheiten mit den Indices b, c und n statistisch verteilt, d.h. es muß sich nicht zwingend um Blockcopolymere handeln.

Das Mittel (a) kann weiterhin auch ein oder mehrere verschiedene aminofunktionalisierte Silikonpolymere enthalten, die durch die Formel (Si-VI)

M(RₐQ_{b}SiO_{(4-a-b)/2)x}(R_{c}SiO_{(4-c)/2)y}M (Si-VI)

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH ₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂ )_{z}NH₂, worin z 1 oder mehr ist. Eine andere mögliche Formel für Z ist -NH(CH₂)_{z}(CH ₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH ₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH ₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO_{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Im Rahmen einer besonders bevorzugten Ausführungsform ist ein erfindungsgemäßes Verfahren gekennzeichnet durch die Anwendung eines Mittels (a) auf dem keratinischem Material, wobei das Mittel (a) ein aminofunktionelles Silikonpolymer der Formel (Si-VII)

R'ₐG₃₋ₐ-Si(OSiG₂)ₙ-(OSiG_{b}R'₂-_{b})ₘ-O-SiG₃₋ₐ-R'ₐ (Si-VII),

enthält, worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃, - CH₂CH₂CH₂CH₃, -O-CH₂CH(CH₃)₂, -CH₂CH(CH₃)₂, -O-CH(CH₃)CH₂CH₃, - CH(CH₃)CH₂CH₃, -O-C(CH₃)₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ∘ -Q-N(R")-CH₂-CH₂-N(R")₂
   ∘ -Q-N(R")₂
   ∘ -Q-N'(R")₃A⁻
   ∘ -Q-N⁺H(R")₂ A⁻
   ∘ -Q-N'H₂(R")A⁻
   ∘ -Q-N(R")-CH₂-CH₂-N'R"H₂A⁻,

wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, - CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Im Rahmen einer weiteren bevorzugten Ausführungsform ist ein erfindungsgemäßes Verfahren gekennzeichnet durch die Anwendung eines Mittels (a) auf dem keratinischem Material, wobei das Mittel (a) mindestens ein aminofunktionelles Silikonpolymer (a1) der Formel (Si-Vlla) enthält, 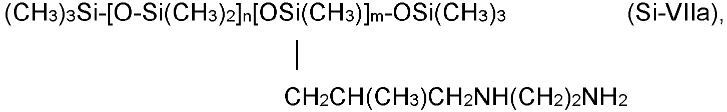 worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Im Rahmen einer weiteren bevorzugten Ausführungsform ist ein erfindungsgemäßes Verfahren gekennzeichnet durch die Anwendung eines Mittels (a) auf dem keratinischem Material, wobei das Mittel (a) mindestens ein aminofunktionelles Silikonpolymer der Formel (Si-Vllb) enthält 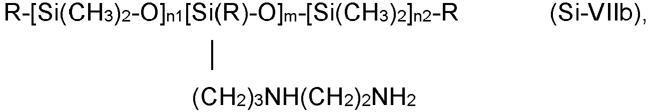 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese aminofunktionalisierten Siliconpolymere werden nach der INCI-Deklaration als Amodimethicone bezeichnet.

Unabhängig davon, welche aminofunktionellen Silicone eingesetzt werden, sind erfindungsgemäße Mittel (a) bevorzugt, die ein aminofunktionelles Silikonpolymer enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Weiterhin sind auch Mittel (a) zum Einsatz im erfindungsgemäßen Verfahren geeignet, welche ein spezielles 4-Morpholinomethyl-substituiertes Silikonpolymer (a1) enthielten. Dieses aminofunktionalisierte Silikonpolymer umfasst Struktureinheiten der Formeln (SI-VIII) und der Formel (Si-IX)

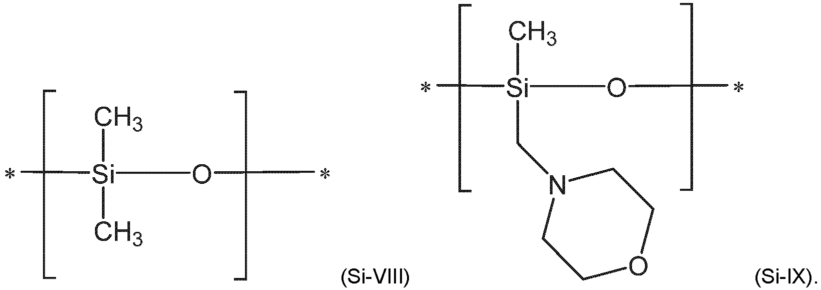

.Entsprechende 4-Morpholinomethyl-substituiertes Silikonpolymere werden im folgenden beschrieben.

Ein ganz besonders bevorzugtes aminofunktionaliserte Silikonpolymer ist unter dem Namen Amodimethicone/Morpholinomethyl Silsesquioxane Copolymer bekannt und in Form des Rohstoffes Belsil ADM 8301 E von Wacker kommerziell erhältlich.

Als 4-morpholinomethyl-substituiertes Silikon kann beispielsweise ein Silikon eingesetzt werden, welches Struktureinheiten der Formeln (Si-VIII), (Si-IX) und (Si-X) aufweist 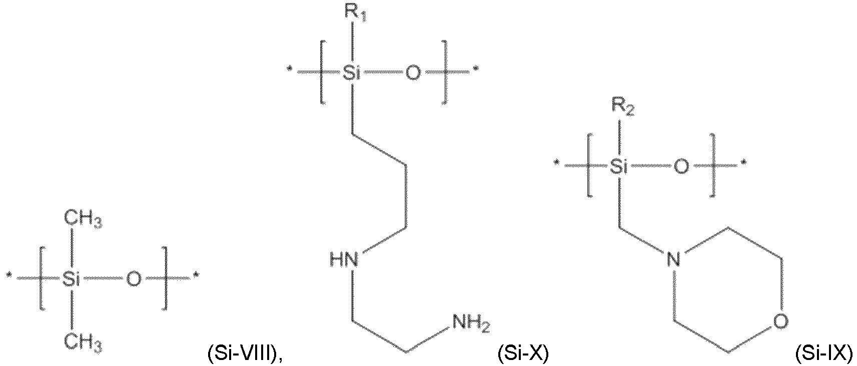 in denen
R1 für -CH₃, -OH, -OCH₃, -O-CH₂CH₃, -O-CH₂CH₂CH₃, oder -O-CH(CH₃)₂ steht;
R2 für -CH₃, -OH, oder -OCH₃ steht.

Besonders bevorzugte erfindungsgemäße Mittel (a) enthalten mindestens ein 4-morpholinomethylsubstituierten Silikons der Formel (Si-XI) 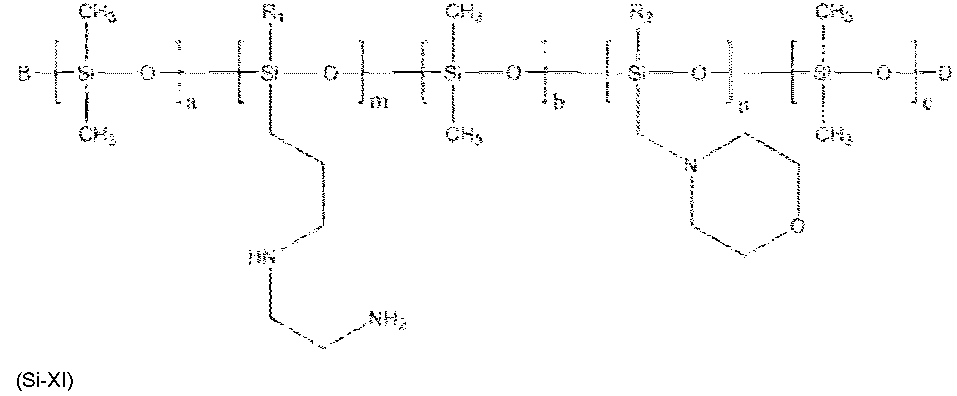 in der
R1 für -CH₃, -OH, -OCH₃, -O-CH₂CH₃, -O-CH₂CH₂CH₃, oder -O-CH(CH₃)₂ steht;
R2 für -CH₃, -OH, oder -OCH₃ steht.
B für eine Gruppe -OH, -O-Si(CH₃)₃,-O-Si(CH₃)₂OH ,-O-Si(CH₃)₂OCH₃ steht,
D für eine Gruppe -H, -Si(CH₃)₃,-Si(CH₃)₂OH, -Si(CH₃)₂OCH₃ steht, a, b und c unabhängig voneinander für ganze Zahlen zwischen 0 und 1000 stehen, mit der Maßgabe a + b + c > 0
m und n unabhängig voneinander für ganze, Zahlen zwischen 1 und 1000 stehen mit den Maßgabe, daß
   - mindestens eine der Bedingungen B = -OH bzw. D = -H erfüllt ist,
   - die Einheiten a, b, c, m und n statistisch oder blockweise im Molekül verteilt vorliegen.

Strukturformel (Si-XI) soll verdeutlichen, daß die Siloxangruppen n und m nicht zwingend direkt an eine Endgruppierung B bzw. D gebunden sein müssen. Vielmehr gilt in bevorzugten Formeln (Si-VI) a > 0 oder b > 0 und in besonders bevorzugten Formeln (Si-VI) a > 0 und c > 0, d.h. die terminale Gruppierung B bzw. D ist vorzugsweise an eine Dimethylsiloxy-Gruppierung gebunden. Auch in Formel (Si-VI) sind die Siloxaneinheiten a, b, c, m und n vorzugsweise statistisch verteilt.

Die durch Formel (Si-VI) dargestellten erfindungsgemäß eingesetzten Silikone können trimethylsilylterminiert sein (D oder B = -Si(CH₃)₃), sie können aber auch zweiseitig dimethylsilylhydroxy- oder einseitig dimethylsilylhydroxy- und dimethylsilylmethoxy-terminiert sein. Im Rahmen der vorliegenden Erfindung besonders bevorzugt eingesetzte Silikone sind ausgewählt aus Silikonen, in denen

| | | |
|---|---|---|
| B = -O-Si(CH₃)₂OH und | D = -Si(CH₃)₃ | |
| B = -O-Si(CH₃)₂OH und | D = -Si(CH₃)₂OH | |
| B = -O-Si(CH₃)₂OH und | D = -Si(CH₃)₂OCH₃ | |
| B = -O-Si(CH₃)₃ | und | D = -Si(CH₃)₂OH |
| B = -O-Si(CH₃)₂OCH₃ | und | D = -Si(CH₃)₂OH |

bedeutet. Diese Silikone führen zu exorbitanten Verbesserungen der Haareigenschaften der mit den erfindungsgemäßen Mitteln behandelten Haare, und zu einem gravierend verbesserten Schutz bei oxidativer Behandlung.

Zur Erzeugung besonders widerstandsfähiger Filme enhält das Mittel (a) das oder die aminofunktionalisierten Silikonpolymere (a2), bevorzugt in bestimmten Mengenbereichen.

Besonders robuste Film wurden erhalten, wenn im erfindungsgemäßen Verfahren ein Mittel (a) eingesetzt wurde, welches - bezogen auf das Gesamtgewicht des Mittels (a) - ein oder mehrere Silikonpolymere in einer Gesamtmenge von 0,1 bis 8,0 Gew.-%, bevorzugt 0,02 bis 5,0 Gew.-%, weiter bevorzugt von 0,1 bis 3,0 Gew.-% und ganz besonders bevorzugt von 0,05 bis 3,5 Gew.-% enthält.

Im Rahmen einer weiteren bevorzugten Ausführungsform ist ein erfindungsgemäßes Verfahren dadurch gekennzeichnet, dass das Mittel (a) - bezogen auf das Gesamtgewicht des Mittels (a) - ein oder mehrere aminofunktionalisierte Silikonpolymere in einer Gesamtmenge von 0,1 bis 8,0 Gew.-%, bevorzugt 0,02 bis 5,0 Gew.-%, weiter bevorzugt von 0,1 bis 3,0 Gew.-% und ganz besonders bevorzugt von 0,05 bis 3,5 Gew.-% enthält.

### farbgebende Verbindung (a2) im Mittel (a)

Als zweiten erfindungswesentlichen Bestandteil enthält das im erfindungsgemäßen Verfahren eingesetzte Mittel (a) mindestens eine farbgebende Verbindung (a2) aus der Gruppe der anorganischen und/oder organischen Pigmente.

Unter farbgebenden Verbindungen werden im Sinne der Erfindung Substanzen verstanden, die in der Lage sind, dem Keratinmaterial eine Färbung zu verleihen. Die farbgebenden Verbindungen werden ausgewählt aus der Gruppe der Pigmente.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente sind anorganischen und/oder organischen Ursprungs.

Ein erfindungsgemäßes Mittel (a) ist dadurch gekennzeichnet, dass es mindestens eine farbgebende Verbindung (a2) aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Verfahren dadurch gekennzeichnet, dass das Mittel (a) mindestens eine farbgebende Verbindung (a2) aus der Gruppe der anorganischen Pigmente enthält, das ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Mittel dadurch gekennzeichnet, dass es (a) mindestens eine farbgebende Verbindung (a2) aus der Gruppe der Pigmente enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} von der Firma Eckart Cosmetic Colors und Sunshine^{®} von der Firma Sunstar erhältlich.

Ganz besonders bevorzugte Farbpigmente mit der Handelsbezeichnung Colorona^{®} sind beispielsweise:
Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491 (Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona^{®} sind beispielsweise:
Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure^{®} beispielsweise:
Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das erfindungsgemäße Mittel auch (a) ein oder mehrere farbgebende Verbindungen (a2) aus der Gruppe der organischen Pigmente enthalten

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Verfahren dadurch gekennzeichnet, dass das Mittel (a) mindestens eine farbgebende Verbindungen (a2) aus der Gruppe der organischen Pigmente enthält, die ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem Mittel (a) des erfindungsgemäßen Verfahrens ganz besonders bevorzugt. Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße D₅₀ von 1,0 bis 50 µm, vorzugsweise von 5,0 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

Das oder die Pigmente können in einer Menge von 0,001 bis 20 Gew.-%, insbesondere von 0,05 bis 5 Gew.-%, jeweils bezogen auf das Gesamtgewicht des Mittels (a), eingesetzt werden.

### filmbildendes Polymer (a3) im Mittel (a)

Bei Anwendung des erfindungsgemäßen Verfahrens besteht die Maßgabe, dass mindestens eines der Mittel (a) und/oder (b) mindestens ein filmbildendes Polymer enthält.

Enthält das Mittel (a) mindestens ein filmbildendes Polymer, so wird dies im Folgenden als (a3) bezeichnet. Enthält das Mittel (b) mindestens ein filmbildendes Polymer, so wird dies im Folgenden als (b2) bezeichnet.

Es ist besonders bevorzugt, wenn das Mittel (a) mindestens ein filmbildendes Polymer (a3) enthält. Es ist ganz besonders bevorzugt, wenn das Mittel (a) mindestens ein hydrophiles, filmbildendes Polymer (a3) enthält. Das filmbildende Polymer (a3) ist von den aminofunktionalisiertes Silikonpolymeren (a1) verschieden.

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen. Bei den Polymeren der vorliegenden Erfindung kann es sich um synthetisch hergestellte Polymere handeln, die durch Polymerisation eines Monomertyps oder durch Polymerisation verschiedener, strukturell voneinander unterschiedlicher Monomertypen hergestellt werden. Wird das Polymer durch Polymerisation eines Monomertyps hergestellt, spricht man von Homo-Polymeren. Werden strukturell unterschiedliche Monomertypen in der Polymerisation eingesetzt, wird das resultierende Polymer als Copolymer bezeichnet.

Das maximale Molekulargewicht des Polymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des filmbildenden, hydrophoben Polymers (c) nicht mehr als 10⁷ g/mol, bevorzugt nicht mehr als 10⁶ g/mol und besonders bevorzugt nicht mehr als 10⁵ g/mol beträgt.

Unter einem hydrophilen Polymer wird ein Polymer verstanden, dass eine Löslichkeit in Wasser bei 25 °C (760 mmHg) von mehr als 1 Gew.-%, bevorzugt von mehr als 2 Gew.-%, besitzt.

Die Wasserlöslichkeit des filmbildenden, hydrophilen Polymers kann beispielsweise auf dem folgenden Weg bestimmt werden. 1,0 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Ein vollständig gelöstes Polymer erscheint markoskopisch homogen. Sofern sich die Polymer-Wasser-Mischung aufgrund einer hohen Trübung des Gemisches nicht visuell beurteilten lässt, wird die Mischung filtriert. Bleibt auf dem Filterpapier kein ungelöstes Polymer zurück, dann liegt die Löslichkeit des Polymers bei mehr als 1 Gew.-%.

Unter einem filmbildenden Polymer wird im Sinne der Erfindung ein Polymer verstanden, welches in der Lage ist, auf einem Substrat, beispielsweise auf einem keratinischen Material oder einer keratinischen Faser, einen Film auszubilden. Die Ausbildung eines Films kann beispielsweise durch Betrachtung des mit dem Polymer behandelten Keratinmaterials unter einem Mikroskop nachgewiesen werden.

Als filmbildende, hydrophile Polymere können nichtionische, anionische und kationische Polymere eingesetzt werden. Als ganz besonders bevorzugt hat sich der Einsatz von nichtionischen, filmbildenden, hydrophilen Polymeren (a3) erwiesen.

In einer weiteren ganz besonders bevorzugten Ausführungsform ist ein erfindungsgemäßes Verahren dadurch gekennzeichnet, dass das Mittel (a) mindestens ein nichtionisches, filmbildendes, hydrophiles Polymer (a3) enthält.

Geeignete filmbildende, hydrophile Polymere können beispielsweise aus der Gruppe der Polyvinylpyrrolidon-(Co)Polymere, der Polyvinylalkohol-(Co)Polymere, der Vinylacetat-(Co)Polymere, der Carboxyvinyl-(Co)Polymere, der Acrylsäure-(Co)Polymere, der Methacrylsäure-(Co)Polymere, der natürlichen Gummen, der Polysaccharide und/oder der Acrylamid-(Co)Polymere ausgewählt werden.

Weiterhin ist es ganz besonders bevorzugt, als filmbildendes hydrophiles Polymer Polyvinylpyrrolidon (PVP) und/oder ein Vinylpyrrolidon-haltiges Copolymer einzusetzen.

In einer weiteren ganz besonders bevorzugten Ausführungsform ist ein erfindungsgemäßes Verahren dadurch gekennzeichnet, dass das Mittel (a) mindestens ein filmbildendes Polymer (a3) enthält, das ausgewählt ist aus der Gruppe aus Polyvinylpyrrolidon (PVP) und den Copolymeren des Polyvinylpyrrolidons.

In einer weiteren ganz besonders bevorzugten Ausführungsform ist ein erfindungsgemäßes Verahren dadurch gekennzeichnet, dass das Mittel (a) mindestens ein filmbildendes, hydrophiles Polymer (a3) enthält, das ausgewählt ist aus der Gruppe aus Polyvinylpyrrolidon (PVP) und den Copolymeren des Polyvinylpyrrolidons.

Es ist explizit am allermeisten bevorzugt, wenn das erfindungsgemäße Mittel als filmbildendes, hydrophiles Polymer (a3) Polyvinylpyrrolidon (PVP) enthält. Überraschenderweise war die Waschechtheit der Färbungen, die mit mit PVP-haltigen Formulierungen erhalten werden konnten, sehr gut.

Besonders gut geeignete Polyvinylpyrrolidone sind beispielsweise unter der Bezeichnung Luviskol^{®} K von der BASF SE erhältlich, insbesondere Luviskol^{®}K 90 oder Luviskol^{®} K 85 der Firma BASF SE.

Als weiteres explizit ganz besonders gut geeignetes Polyvinylpyrrolidon (PVP) kann auch das Polymer PVP K30 eingesetzt werden, das von der Firma Ashland (ISP, POI Chemical) vertrieben wird. PVP K 30 ist ein in kaltem Wasser sehr gut lösliches Polyvinylpyrrolidon, welches die CAS-Nummer 9003-39-8 besitzt. Das Molgewicht von PVP K 30 liegt bei ca. 40000 g/mol.

Weitere ganz besonders gut geeignete Polyvinylpyrrolidone sind die unter den Handelsnamen LUVITEC K 17, LUVITEC K 30, LUVITEC K 60, LUVITEC K 80, LUVITEC K 85, LUVITEC K 90 und LUVITEC K 115 bekannten und von der BASF erhältlichen Substanzen.

Der Einsatz von filmbildenden hydrophilen Polymeren (a3) aus der Gruppe der Copolymere des Polyvinylpyrrolidons hat ebenfalls zu besonders guten und waschechten Farbergebnissen geführt. Auch die Lagerstabilitäten der Formulierungen, die ein oder mehrere Copolymere des Polyvinylpyrrolidons (a3) enthielten, waren sehr gut.

Als besonders gut geeignete filmbildende, hydrophile Polymere können in diesem Zusammenhang Vinylpyrrolidon-Vinylester-Copolymere genannt werden, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind besonders bevorzugte nichtionische Polymere.

Von den Vinylpyrrolidon-haltigen Copolymeren werden ganz besonders bevorzugt ein Styrene/VP Copolymer und/oder ein Vinylpyrrolidon-Vinylacetat-Copolymer und/oder ein VP/DMAPA Acrylates Copolymer und/oder ein VP/Vinyl Caprolactam/DMAPA Acrylates Copolymer in den kosmetischen Zusammensetzungen eingesetzt.

Vinylpyrrolidon-Vinylacetat-Copolymere werden unter der Bezeichnung Luviskol^{®} VA von der BASF SE vertrieben. Ein VP/Vinyl Caprolactam/DMAPA Acrylates Copolymer wird beispielsweise unter dem Handelsnamen Aquaflex^{®} SF-40 von Ashland Inc. vertrieben. Ein VP/DMAPA Acrylates Copolymer wird beispielsweise unter der Bezeichnung Styleze CC-10 von Ashland vertrieben und ist ein höchst bevorzugtes Vinylpyrrolidon-haltiges Copolymer.

Als weitere geeignete Copolymere des Polyvinylpyrrolidons (c) können auch die Copolymere genannt werden, die durch Umsetzung von N-Vinylpyrrolidon mit mindestens einem weiteren Monomer aus der Gruppe aus V-Vinylformamid, Vinylacetat, Ethylen, Propylen, Acrylamid, Vinylcaprolactam, Vinylcaprolacton und/oder Vinylalkohol erhalten werden.

In einer weiteren ganz besonders bevorzugten Ausführungsform ist ein erfindungsgemäßes Verfahren gekennzeichnet, dass das Mittel (a) mindestens ein filmbildendes Polymer (a3) enthält, das ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/Ethylen-Copolymeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren, explizit ganz besonders bevorzugt Polyvinylpyrrolidon (PVP).

Ein weiteres geeigetes Copolymer des Vinylpyrrolidons ist das unter der INCI Bezeichnung Maltodextrin/VP Copolymer bekannte Polymer.

Weiterhin konnte intensiv gefärbtes Keratinmaterial, insbesondere Haare, mit sehr guten Waschechtheiten erhalten werden, wenn als filmbildendes, hydrophiles Polymer ein nichtionisches, filmbildendes, hydrophiles Polymer eingesetzt wurde.

In einer ganz besonders bevorzugten Ausführungsform ist ein erfindungsgemäßes Mittel (a) dadurch gekennzeichnet, dass es mindestens ein nichtionisches, filmbildendes, hydrophiles Polymer (a3) enthält.

Unter einem nichtionischen Polymer wird erfindungsgemäß ein Polymer verstanden, das in einem protischen Lösemittel - wie beispielsweise Wasser - bei Standardbedingungen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Es sind die Mittel ganz besonders bevorzugt, die als nichtionisches, filmbildendes, hydrophiles Polymer mindestens ein Polymer enthalten, das ausgewählt ist aus der Gruppe aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymeren aus N-Vinylpyrrolidon und N-Vinylimidazol und Methacrylamid,
- Copolymeren aus N-Vinylpyrrolidon und N-Vinylimidazol und Acrylamid,
- Copolymeren aus N-Vinylpyrrolidon mit N,N-Di(C₁ bis C₄)-Alkylamino-(C₂ bis C₄)-alkylacrylamid.

Kommen Copolymere aus N-Vinylpyrrolidon und Vinylacetat zum Einsatz, ist es wiederum bevorzugt, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt. Geeignete Copolymerisate aus Vinylpyrrolidon und Vinylacetat sind beispielsweise unter dem Warenzeichen Luviskol^{®} VA 37, Luviskol^{®} VA 55, Luviskol^{®} VA 64 und Luviskol^{®} VA 73 von der Firme BASF SE erhältlich.

Ein weiteres besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Ein weiteres ganz besonders bevorzugtes nichtionisches, filmbildendes, hydrophiles Polymer st ein Copolymer aus N-Vinylpyrrolidon und N,N-Dimethylaminiopropylmethacrylamid,welches beispielsweise mit der INCI-Bezeichnung VP/DMAPA Acrylates Copolymer z. B. unter der Handelsbezeichnung Styleze^{®} CC 10 von der Firma ISP verkauft wird.

Ein kationisches erfindungsgemäßes Polymer ist das Copolymer aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle^{®} 300 (28-32 Gew.-% Aktivsubstanz in Ethanol-WasserGemisch, Molekulargewicht 350000) von der Firma ISP vertrieben wird.

Weitere geeignete filmbildende, hydrophile Polymere sind beispielsweise
- Vinylpyrrolidon-Vinylimidazoliummethochlorid-Copolymere, wie sie unter den Bezeichnungen Luviquat^{®} FC 370, FC 550 und der INCI-Bezeichnung Polyquaternium-16 sowie FC 905 und HM 552 angeboten werden,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Polyquaternium-11 ist das Reaktionsprodukt von Diethylsulfat mit einem Copolymer von Vinylpyrrolidon und Dimethylaminoethylmethacrylat. Geeignete Handelsprodukte sind beispielsweise unter den Bezeichnungen Dehyquart^{®} CC 11 und Luviquat^{®} PQ 11 PN von der BASF SE oder Gafquat 440, Gafquat 734, Gafquat 755 oder Gafquat 755N von Ashland Inc. erhältlich.

Polyquaternium-46 ist das Reaktionsprodukt von Vinylcaprolactam und Vinylpyrrolidon mit Methylvinylimidazoliummethosulfat und ist beispielsweise unter der Bezeichnung Luviquat^{®} Hold von der BASF SE erhältlich. Polyquaternium-46 wird bevorzugt in einer Menge von 1 bis 5 Gew.-% - bezogen auf das Gesamtgewicht der kosmetischen Zusammensetzung - eingesetzt. Es ganz besonders bevorzugt, dass Polyquaternium-46 in Kombination mit einer kationischen Guar-Verbindung eingesetzt wird. Es ist sogar höchst bevorzugt, dass Polyquaternium-46 in Kombination mit einer kationischen Guar-Verbindung und Polyquaternium-11 eingesetzt wird.

Als geeignete anionische filmbildende, hydrophile Polymere können zum Beispiel Acrylsäure-Polymere eingesetzt werden, die in unvernetzter oder vernetzter Form vorliegen können. Entsprechende Produkte werden beispielsweise unter den Handelsnamen Carbopol 980, 981, 954, 2984 and 5984 von der Firma Lubrizol oder auch unter den Namen Synthalen M and Synthalen K von der Firma 3V Sigma (The Sun Chemicals, Inter Harz) kommerziell vertrieben.

Beispiele für geeignete filmbildende, hydrophile Polymere aus der Gruppe der natürlichen Gums sind Xanthan Gum, Gellan Gum, Carob Gum .

Beispiele für geeignete filmbildende, hydrophile Polymere aus der Gruppe der Polysaccharide sind Hydroxyethylcellulose, Hydroxypropylcellulose, Ethylcellulose und Carboxymethyl-Cellulose.

Geeignete filmbildende, hydrophile Polymere aus der Gruppe der Acrylamide sind beispielsweise Polymere, welche ausgehend von Monomeren der (Methy)Acrylamido-C1-C4-alkyl-sulfonsäure bzw. der Salze hiervon hergestellt werden. Entsprechende Polymere können ausgewählt sein aus den Polymeren von Polyacrylamidomethansulfonsäure, Polyacrylamidoethansulfonsäure, Polyacrylamidopropansulfonsäure, Poly2-acrylamido-2-methylpropansulfonsäure, Poly-2-Methylacrylamido-2-methylpropansulfonsäure und/oder Poly-2-methylacrylamido-n-butansulfonsäure.

Bevorzugte Polyemre der Poly(meth)arylamido-C1-C4-alkyl-sulfonsäuren sind vernetzt und zu mindestens 90 % neutralisiert. Diese Poylmere können vernetzt oder auch unvernetzt sein.

Vernetzte und ganz oder teilweise neutraliserte Poylmere des Typs der Poly-2-acrylamido-2-methylpropansulfonsäuren sind unter den INCI-Namen "Ammonium Polyacrylamido-2-methyl-propanesulphonate" oder "Ammonium Polyacryldimethyltauramide" bekannt.

Ein weiteres bevorzugtes Polymer dieses Typs ist das der Firma Clamant unter dem Handelsnamen Hostacerin AMPS vertriebene vernetzte Poly-2-acrylamido-2methyl-propanesulphonsäure-Polymer, das teilweise mit Ammoniak neutralisiert ist.

Das oder die erfindungsgemäßen filmbildenden Polymere (a3) werden bevorzugt in bestimmten Mengenbereichen im erfindungsgemäßen Mittel eingesetzt. In diesem Zusammenhang hat es sich zur Lösung der erfindungsgemäßen Aufgabenstellung als besonders bevorzugt erwiesen, wenn das Mittel (a) - bezogen auf das Gesamtgewicht des Mittels (a) - ein oder mehrere filmbildende Polymere (a3) in einer Gesamtmenge von 0,1 bis 25,0 Gew.-%, bevorzugt von 0,2 bis 20,0 Gew-%, weiter bevorzugt von 0,5 bis 15,0 Gew.-% und ganz besonders bevorzugt von 1,0 bis 7,0 Gew.-% enthält.

### pH-Wert des Mittels (a)

Das Färbemittel (a) wird bevorzugt auf einen neutral bis alkalischen pH-Wert eingestellt. Ganz besonders bevorzugt wird das Färbemittel (a) auf einen alkalischen pH-Wert eingestellt. Unter basischen Bedingungen kann das aminofunktionalisierte Silikonpolymer (a1) besonders gut und ohne Protonierung gelöst bzw. dispergiert werden.

Zur Einstellung des gewünschten pH-Wertes enthält das Mittel (a) bevorzugt mindestens ein Alkalisierungsmittel enthalten. Bei den pH-Werten im Sinne der vorliegenden Erfindung handelt es sich um pH-Werte, die bei einer Temperatur von 22°C gemessen wurden.

Als Alkalisierungsmittel kann das Mittel (a) beispielsweise Ammoniak, Alkanolamine und/oder basische Aminosäuren enthalten.

Die in dem erfindungsgemäßen Mittel einsetzbaren Alkanolamine werden bevorzugt ausgewählt aus primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

Erfindungsgemäß besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass das erfindungsgemäße Mittel als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindestens eine protonierbare Aminogruppe und mindestens eine -COOH- oder eine -SO₃H-Gruppe enthält. Bevorzugte Aminosäuren sind Aminocarbonsäuren, insbesondere α-(alpha)-Aminocarbonsäuren und ω-Aminocarbonsäuren, wobei α-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus kann das Mittel weitere Alkalisierungsmittel, insbesondere anorganische Alkalisierungsmittel enthalten. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

In einer weiteren ganz besonders bevorzugten Ausführungsform ist ein erfindungsgemäßes Verfahren dadurch gekennzeichnet, dass das Färbemittel (a) mindestens ein Alkalisierungsmittel aus der Gruppe aus Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methyl-propan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat enthält.

Besonders gute Ergebnisse wurden erhalten, wenndas Mittel (a) auf einen pH-Wert 7,0 bis 11,5 bevorzugt von 8,0 bis 11,0, und besonders bevorzugt von 8,5 bis 10,5 eingestellt wurde.

In einer weiteren ganz besonders bevorzugten Ausführungsform ist ein erfindungsgemäßes Verfahren dadurch gekennzeichnet, dass das Mittel (a) Wasser enthält und einen pH-Wert von 7,0 bis 11,5 bevorzugt von 8,0 bis 11,0, und besonders bevorzugt von 8,5 bis 10,5 besitzt.

### Säure (b1) im Mittel (b)

Im Anschluss an die Anwendung des Färbemittels (a) auf dem Keratinmaterial wird das Nachbehandlungsmittel (b) angewendet. Bei dem Nachbehandlungsmittel (b) handelt es sich um eine sauer eingestellte Lösung, Dispersion oder Emulsion. Als erfindungswesentliche Inhaltsstoffe enthält das Nachbehandlungsmittel (b) deshalb Wasser und mindestens eine Säure (b1) und besitzt einen pH-Wert von 1,5 bis 5,5.

Hierbei haben sich bestimmte Säuren zur Einstellung des gewünschten pH-Wertes als ganz besonders gut geeignet erwiesen. Diese Säuren können beispeilsweise ausgewählt werden aus der Gruppe aus Zitronensäure, Weinsäure, Äpfelsäure, Milchsäure, Essigsäure, Methansulfonsäure, Benzoesäure, Malonsäure, Oxalsäure, 1-Hydroxyethan-1,1-diphosphonsäure, Schwefelsäure, Salzsäure und Phosphorsäure.

In einer weiteren ganz besonders bevorzugten Ausführungsform ist ein erfindungsgemäßes Verfahren dadurch gekennzeichnet, dass das Mittel (b) mindestens eine Säure (b1) enthält, die ausgewählt ist aus der Gruppe aus Zitronensäure, Weinsäure, Äpfelsäure, Milchsäure, Essigsäure, Methansulfonsäure, Benzoesäure, Malonsäure, Oxalsäure, 1-Hydroxyethan-1,1-diphosphonsäure, Schwefelsäure, Salzsäure und Phosphorsäure.

Sobald das Nachbehandlungsmittel (b) angewendet wird, tritt dieses mit den auf dem Keratinmaterial abgelagerten Aminosilikonen (a1) und farbgebenden Verbindungen (a2) in Kontakt. Da das Mittel (b) sauer eingestellt ist, senkt es auch den pH-Wert in der direkten Umgebung des Aminosilikons (a1) ab. In diesem Zusammenhang wird vermutet, dass durch die Verringerung des pH-Wertes eine Protonierung des Aminosilikons (a1) erfolgt, wodurch in Folge dessen Adhäsionskräfte zum Keratinmaterial weiter verstärkt und die farbgebenden Verbindungen (a2) noch stärker an das Haar gebunden werden. Hierdurch bedingt wird die Waschechtheit der resultierenden Färbung massiv verbessert. Aus diesem Grund wird das Nachbehandlungsmittel (b) auf einen sauren pH-Wert im Bereich von 1,5 bis 5,5, bevorzugt von 2,0 bis 4,8, und besonders bevorzugt von 2,5 bis 4,5 eingestellt.

In einer weiteren ganz besonders bevorzugten Ausführungsform ist ein erfindungsgemäßes Verfahren dadurch gekennzeichnet, dass das Mittel (b) Wasser enthält und einen pH-Wert von 2,0 bis 4,8, und bevorzugt von 2,5 bis 4,5 besitzt.

Im Rahmen der zu dieser Erfindung führenden Arbeiten hat sich die Wahl des Zeitpunktes, zu dem der pH-Wert abgesenkt wird, als wesentlich für die Erzeugung von stabilen Schichten auf dem Keratinmaterial herausgestellt. Es ist erfindungswesentlich, dass der pH-Wert des Aminosilikons (a1) abgesenkt wird, nachdem dieses auf die Keratinfaser aufgetragen wurde. Bei zeitgleicher Anwendung von Aminosilikon (a1) und Säure findet keine signifikante Verstärkung der Bindung an das Keratin statt. Aus diesem Grund ist es erfindungswesentlich, die Säure (b1) in das Nachbehandlungsmittel (b) einzuarbeiten.

Es wurde gefunden, dass die Verbesserung der Waschechtheit umso größer ist, je stärker der pH-Wert in der Umgebung des Aminosilikons (a1) abgesenkt wird. Mit anderen Worten waren die Färbungen insbesondere dann besonders beständig, wenn das Färbemittel (a) vergleichsweise stark alkalisch eingestellt war, und das Nachbehandlungsmittel (b) einen verhältnismäßig sauren pH-Wert besaß.

### filmbildendes Polymer (b2) im Mittel (b)

Bei dem erfindungsgemäßen Verfahren besteht die Maßgabe, dass mindestens eine der im Verfahren eingesetzten Mittel (a) und/oder (b) ein filmbildendes Polymer enthält. Wenn das filmbildende Polymer im Nachbehandlungsmittel (b) enthalten ist, wird dieses als (b2) bezeichnet.

Die im Mittel (b) enthaltenen filmbildenden Polymere können dieselben sein, die bereits im Zusammenhang mit dem Mittel (a) beschrieben wurden.

So kann das Nachbehandlungsmittel (b) beispielsweise ein filmbildendes Polymer (b2) enthalten, das ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/Ethylen-Copolymeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren, explizit ganz besonders bevorzugt Polyvinylpyrrolidon (PVP).

Darüberhinaus kann das Nachbehandlungsmittel (b) auch ein filmbildendes Polymer enthalten, das augewählt ist aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Zur Lösung der erfindungsgemäßen Aufgabenstellung haben sich insbesondere die filmbildenden Polymere als gut geeignet erwiesen, die aus der Gruppe der synthetischen Polymere, der durch radikalische Polymerisation erhältlichen Polymere oder der natürlichen Polymere ausgewählt werden.

Weitere besonders gut geeignete filmbildende Polymere können ausgewählt werden aus den Homopolymere oder Copolymeren von Olefinen, wie beispielsweise Cycloolefinen, Butadien, Isopren oder Styren, Vinylethern, Vinylamiden, den Estern oder Amiden von (Meth)Acrylsäure mit mindestens einer C₁-C₂₀-Alkylgruppe, einer Arylgruppe oder einer C2-C10-Hydroxyalkylgruppe.

Weitere filmbildende Polymere können ausgewählt sein aus den Homo- oder Copolymeren von Isooctyl-(meth)acrylat; Isononyl-(meth)acrylat; 2-Ethylhexyl-(meth)acrylat; Lauryl-(meth)acrylat); isopentyl-(meth)acrylat; n-Butyl-(meth)acrylat); Isobutyl-(meth)acrylat; Ethyl-(meth)acrylat; Methyl-(meth)acrylat; tert-Butyl-(meth)acrylat; Stearyl-(meth)acrylat; Hydroxyethyl-(meth)acrylat; 2-Hydroxypropyl-(methacrylat; 3-Hydroxypropyl-(meth)acrylat und/oder Gemischen hiervon.

Weitere filmbildende Polymere können ausgewählt sein aus den Homo- oder Copolymeren von (Meth)acrylamid; N-Alkyl-(meth)acrylamiden, insbesondere solchen mit C2-C18 Alkylgruppen, wie beispielsweise N-Ethyl-acrylamid, N-tert-butyl-acrylamid, le N-Octyl-crylamid; N-Di(C1-C4)alkyl-(meth)acrylamid.

Weitere bevorzugte anionische Copolymere sind beispielsweise Copolymere aus Acrylsäure, Methacrylsäure oder deren C₁-C₆-Alkylestern, wie sie unter der INCI-Deklaration Acrylates Copolymere vertrieben werden. Ein geeignetes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁-C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20.

Auf dem Markt befindliche ganz besonders bevorzugte Polymere sind beispielsweise Aculyn^{®} 22 (Acrylates/Steareth-20 Me-thacrylate Copolymer), Aculyn^{®} 28 (Acrylates/Beheneth-25 Methacrylate Copolymer), Structure 2001^{®} (Acryla-tes/Steareth-20 Itaconate Copolymer), Structure 3001^{®} (Acrylates/Ceteth-20 Itaconate Copolymer), Structure Plus^{®} (Acrylates/Aminoacrylates C10-30 Alkyl PEG-20 Itaconate Copolymer), Carbopol^{®} 1342, 1382, Ultrez 20, Ultrez 21 (Acrylates/C10-30 Alkyl Acrylate Crosspolymer), Synthalen W 2000^{®} (Acrylates/Palmeth-25 Acrylate Copolymer) oder das Rohme und Haas vertriebene Soltex OPT (Acrylates/C12-22 Alkyl methacrylate Copolymer).

Als geeignete Polymere auf der Basis von Vinyl-Monomeren können beispielsweise genannt werden die Homo- und Copolymere des N-Vinylpyrrolidons, des Vinylcaprolactams, des Vinyl-(C1-C6-)alkyl-Pyrrols, des Vinyl-oxazols, des Vinyl-thiazols, des Vinylpyrimidins, des Vinylimidazols.

Weiterhin ganz besonders gut geeignet sind die Copolymere Octylacrylamid/acrylates/ butylaminoethyl-methacrylate copolymer, wie es beispielsweise unter den Handelsnamen AMPHOMER^{®} ou LOVOCRYL^{®} 47 von NATIONAL STARCH kommerziell vertrieben wird, oder auch die Copolymere von Acrylates/Octylacrylamide die unter den Handelsnamen DERMACRYL^{®} LT und DERMACRYL^{®} 79 von NATIONAL STARCH vertrieben werden.

Als geeignete Polymere auf der Basis von Olefinen können beispielsweise genannt werden die Homo- und Copolymere des Ethylens, des Propylens, des Butens, des Isoprens und des Butadiens. Im Rahmen einer weiteren Ausführungsform können als filmbildenden hydrophoben Polymere die Block-Copoylmere eingesetzt werden, die mindestens einen Block aus Styren oder den Derivaten des Styrens umfassen. Bei diesen Block-Copolymeren kann es sich um Copolymere handeln, die neben einem Styren-Block einen oder mehrere weitere Blöcke enthalten, wie beispielsweise Styren/Ethylen, Styren/Ethylen/Butylen, Styen/Butylen, Styren/Isopren, Styren/Butadien. Entsprechende Polymere werden von der BASF unter dem Handelsnamen "Luvitol HSB" kommerziell vertrieben.

In einer weiteren ganz besonders bevorzugten Ausführungsform ist ein erfindungsgemäßes Verfahren dadurch gekennzeichnet, dass das Nachbehandlungsmittel (b) mindestens ein filmbildendes Polymer (b2) enthält, das ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/Ethylen-Copolymeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren, der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Das oder die filmbildenden Polymere werden bevorzugt in bestimmten Mengenbereichen im Nachbehandlungsmittel (b) eingesetzt. In diesem Zusammenhang hat es sich zur Lösung der erfindungsgemäßen Aufgabenstellung als besonders bevorzugt erwiesen, wenn das Mittel (b) - bezogen auf das Gesamtgewicht des Mittels (b) - ein oder mehrere Polymere in einer Gesamtmenge von 0,1 bis 25,0 Gew.-%, bevorzugt von 0,2 bis 20,0 Gew-%, weiter bevorzugt von 0,5 bis 15,0 Gew.-% und ganz besonders bevorzugt von 1,0 bis 7,0 Gew.-% enthält.

### Weitere Inhaltsstoffe in den Mitteln (a) und (b)

Die zuvor beschriebenen Mittel (a) und (b) können ferner auch noch ein oder mehrere optionale Inhaltsstoffe enthalten.

Die Mittel können zusätzlich ein oder mehrere Tenside enthalten. Unter dem Begriff Tenside werden grenzflächenaktive Substanzen verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, Imidazoliniumbetaine und Sulfobetaine.

Besonders bevorzugte ampholytische Tenside sind das N-Kokosalkylaminopropionat, das Kokosacylaminoethylaminopropionat und das C₁₂ - C₁₈ - Acylsarcosin.

Die Mittel können auch zusätzlich mindestens ein nichtionisches Tensid enthalten. Geeignete nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit guten Eigenschaften werden ebenfalls erhalten, wenn sie als nichtionische Tenside Fettsäureester von ethoxyliertem Glycerin enthalten, die mit mindestens 2 Mol Ethylenoxid umgesetzt wurden. Die nichtionischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Weiterhin können die Mittel zusätzlich auch mindestens ein kationisches Tensid enthalten. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Beispiele für kationische Tenside sind
- quartäre Ammoniumverbindungen, die als hydrophobe Reste ein oder zwei Alkylketten mit einer Kettenlänge von 8 bis 28 C-Atomen tragen können,
- quartäre Phosphoniumsalze, substituiert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein. Neben der funktionellen Einheit, welche die kationische Ladung trägt, kann das kationische Tensid auch weitere ungeladene funktionelle Gruppen beinhalten, wie dies beispielsweise bei Esterquats der Fall ist. Die kationischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Weiterhin können die erfindungsgemäßen Mittel auch mindestens ein anionisches Tensid enthalten. Als anionische Tenside werden oberflächenaktive Mittel mit ausschließlich anionischen Ladungen (neutralisiert durch ein entsprechendes Gegenkation) bezeichnet. Beispiele für anionische Tenside sind Fettsäuren, Alkylsulfate, Alkylethersulfate und Ethercarbonsäuren mit 12 bis 20 C-Atomen in der Alkylgruppe und bis zu 16 Glykolethergruppen im Molekül.

Die anionischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Die Mittel können ferner auch noch weitere Wirk-, Hilfs- und Zusatzstoffe enthalten, wie beispielsweise Lösungsmittel, Fettbestandteile wie beispielsweise der C₈-C₃₀-Fettalkohole, der C₈-C₃₀-Fettsäuretriglyceride, der C₈-C₃₀-Fettsäuremonoglyceride, der C₈-C₃₀-Fettsäurediglyceride und/oder der Kohlenwasserstoffe; Polymere; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

### Verfahren zum Färben von Keratinmaterialien

Im Rahmen des erfindungsgemäßen Verfahrens werden die Mittel (a) und (b) auf die keratinischen Materialien, insbesondere auf die menschlichen Haare, appliziert. Das Mittel (b) stellt ein Nachbehandlungsmittel dar und wird demzufolge im Anschluss an das Färbemittel (a) appliziert.

Die Mittel (a) und (b) werden innerhalb ein und desselben Färbeverfahrens angewendet, was bedeutet, dass zwischen der Anwendung der Mittel (a) und (b) ein Zeitraum von maximal einigen Stunden liegt.

Das erfindungsgemäßes Verfahren ist dadurch gekennzeichnet, das zunächst das Mittel (a) angewendet wird, und danach das Mittel (b) angewendet wird, wobei der Zeitraum zwischen der Anwendung der Mittel (a) und (b) bei maximal 24 Stunden, bevorzugt bei maximal 12 Stunden und besonders bevorzugt bei maximal 6 Stunden liegt.

Im Rahmen des erfindungsgemäßen Verfahrens werden die Keratinmaterialien, insbesondere die menschlichen Haare, zunächst mit Färbemittel (a) behandelt. Im Anschluss daran wird das Nachbehandlungsmittel (b) auf die Keratinmaterialien gegeben, welches den pH-Wert auf der Oberfläche des Keratinmaterials absenkt die im Mittel (a) enthaltenen Wirkstoffe auf dem Keratin auf diese Weise fixiert oder immobilisiert. Bevorzugt enthält das Mittel (b) selbst keine Farbstoffe bzw. keine farbgebenden Verbindungen.

Die anwendungstechnischen Eigenschaften der resultierenden Färbung können durch Wahl der optimalen Verfahrensbedingungen noch weiter verbessert werden.

Im Rahmen einer weiteren Ausführungsform ganz besonders bevorzugt ist ein Verfahren, umfassend die folgenden Schritte in der angegebenen Reihenfolge
(1) Anwendung des Färbemittels (a) auf dem keratinischen Material,
(2) Einwirken lassen des Mittels (a) für einen Zeitraum von 10 Sekunden bis 10 Minuten, bevorzugt von 10 Sekunden bis 5 Minuten,
(3) gegebenenfalls Ausspülen des keratinischen Materials mit Wasser,
(4) Anwendung des Nachbehandlungsmittels (b) auf dem keratinischen Material,
(5) Einwirken lassen des Mittels (b) für einen Zeitraum von 30 Sekunden bis 30 Minuten, bevorzugt von 30 Sekunden bis 10 Minuten, und
(6) Ausspülen des keratinischen Materials mit Wasser.

Unter dem Ausspülen des keratinischen Materials mit Wasser in den Schritten (3) und (6) des Verfahrens wird erfindungsgemäß verstanden, dass für den Ausspülvorgang nur Wasser verwendet wird, ohne dass noch weitere, von den Mitteln (a) und (b) verschiedene Mittel zur Anwendung kämen.

In einem Schritt (1) wird zunächst das Mittel (a) auf die Keratinmaterialien, insbesondere die menschlichen Haare, appliziert.

Nach dem Auftragen wird das Mittel (a) auf die Keratinmaterialien einwirken gelassen. Das erfindungsgemäße Verfahren erlaubt selbst bei kurzer Einwirkzeit des Mittels (a) die Erzeugung von Färbungen mit besonders guter Intensität und Waschechtheit. In diesem Zusammenhang haben sich Einwirkzeiten von 10 Sekunden bis 10 Minuten, bevorzugt von 20 Sekunden bis 5 Minuten und ganz besonders bevorzugt von 30 Sekunden bis 2 Minuten auf den Haaren haben sich als besonders vorteilhaft erwiesen.

Im Rahmen einer bevorzugten Ausführungsform des erfindungsgemäßen Verfahrens kann das Mittel (a) nun von den Keratinmaterialien ausgespült werden, bevor das Mittel (b) im nachfolgenden Schritt auf die Haare appliziert wird.

Färbungen mit ebenfalls guten Waschechtheiten wurden erhalten, wenn das Mittel (b) auf die Keratinmaterialien appliziert wurde, die noch mit dem Mittel (a) beaufschlagt waren.

In Schritt (4) wird nun das Mittel (b) auf die Keratinmaterialien appliziert. Nach dem Auftragen wird nun das Mittel (b) auf die Haare einwirken gelassen.

Das erfindungsgemäße Verfahren erlaubt auch bei kurzer Einwirkzeit des Mittels (b) die Erzeugung von Färbungen mit besonders guter Intensität und Waschechtheit. Einwirkzeiten von 10 Sekunden bis 10 Minuten, bevorzugt von 20 Sekunden bis 5 Minuten und ganz besonders bevorzugt von 30 Sekunden bis 3 Minuten auf den Haaren haben sich als besonders vorteilhaft erwiesen.

In Schritt (6) wird nun das Mittel (b) (sowie ggf. noch vorhandenes Mittel (a)) mit Wasser aus dem Keratinmaterial ausgespült.

Im Rahmen einerweiteren Ausführungsform ganz besonders bevorzugt ist ein Verfahren, umfassend die folgenden Schritte in der angegebenen Reihenfolge
(1) Anwendung des Mittels (a) auf dem keratinischen Material,
(2) Einwirken lassen des Mittels (a) für einen Zeitraum von 10 Sekunden bis 10 Minuten, bevorzugt von 10 Sekunden bis 5 Minuten,
(3) kein Ausspülen
(4) Anwendung des Mittels (b) auf dem keratinischen Material,
(5) Einwirken lassen des Mittels (b) für einen Zeitraum von 30 Sekunden bis 30 Minuten, bevorzugt von 30 Sekunden bis 10 Minuten, und
(6) Ausspülen des keratinischen Materials mit Wasser.

In Rahmen dieser Ausführungsform erfolgt die Abfolge der Schritte (1) bis (6) innerhalb von 24 Stunden.

Beispiele

### 1. Formulierungen

Es wurden die folgenden Formulierungen hergestellt (alle Angaben sind, sofern nichts anderes angegeben ist, in Gew.-%)

### Färbemittel (a)

| Mittel (a) | (aV) Vergleich | (aE) Erfindung |
|---|---|---|
| Cetrimoniumchlorid | 3,3 | 3,3 |
| Stearamidopropyldimethylamin | 1,0 | 1,0 |
| Phenoxyethanol | 0,4 | 0,4 |
| Methylparaben | 0,3 | 0,3 |
| Dimethicone 5 cst | 5,0 | 5,0 |
| Dimethicone 60000 cst | 1,0 | 1,0 |
| Luviskol K30 (BASF, Polyvinylpyrrolidon) | 5,0 | 5,0 |
| Lavanya Zuni (organisches Pigment,Neelikon Red, 111P0200, CI 12490) | 0,5 | 0,5 |
| Dow Corning 2-8566 (Siloxanes and Silicones, 3-[(2-Aminoethyl)amino]-2-methylpropyl Me, Di-Me-Siloxane" | 0,50 | 0,50 |
| 1,2-Propandiol | 0,25 | 0,25 |
| Ammoniak | --- | ad pH 9,0 |
| Zitronensäure | ad pH 3,0 | --- |
| Wasser | ad 100 | ad 100 |

### Nachbehandlungsmittel (b)

| Mittel (b) | (bV) Vergleich | (bE) Erfindung |
|---|---|---|
| Zitronensäure | --- | ad pH 3,0 |
| Wasser | ad 100 | ad 100 |

### 2. Anwendung

Das Mittel (a) wurde jeweils auf eine Haarsträhne (Kerling, Euronaturhaar weiß, Flottenverhältnis: 4 g Mittel (a) pro g Haarsträhne) appliziert und dann für eine Minute einwirken gelassen. Danach wurde die Haarsträhne in das Nachbehandlungsmittel (b) getaucht und für 1 Minute darin belassen. Im Anschluss daran wurde jede Haarsträhne gründlich (1 Minute) mit Wasser ausgewaschen, getrocknet und visuell unter der Tageslichtlampe bewertet.

Zur Bestimmung der Waschechtheiten wurden die zuvor gefärbten Haarsträhnen in ein Ultraschallbad gegeben, das mit einer 1 %igen Lösung eines handelsüblichen Shampoos (Schaume, 7 Kräuter) befüllt war. Dann wurden die Haarsträhnen nach einem standardisierten Verfahren, welches 6 Haarwäschen entspricht, mit Ultraschall behandelt. Nach diesem Zeitraum wurden die Strähnen aus dem Ultraschallbad entnommen, getrocknet und erneut visuell unter der Tageslichtlampe beurteilt.

| | Anwendung | Anwendung |
|---|---|---|
| Färbemittel (a) | (aV) | (aE) |
| Nachbehandlungsmittel (b) | (bV) | (bE) |
| Färbung | rötlich | rot |
| Farbintensität (direkt nach der Färbung) | ++ | +++ |
| Farbintgensität (nach 6 Haarwäschen9 | + | +++ |

| | | |
|---|---|---|
| Farbintensität: - = ungefärbt + = gering ++ = mittel +++ = sehr gut | | |

## Patentansprüche

1. Verfahren zum Färben von keratinischem Material, insbesondere menschlichen Haaren, umfassend die folgenden Schritte:
- Anwendung eines Färbemittels (a) auf dem keratinischem Material, wobei das Mittel (a) enthält:
(a1) mindestens ein aminofunktionalisiertes Silikonpolymer, und
(a2) mindestens eine farbgebende Verbindung aus der Gruppe der anorganischen und/oder organischen Pigmente, und
(a3) gegebenenfalls mindestens ein filmbildendes Polymer, das von (a1) verschieden ist,
- Anwendung eines Nachbehandlungsmittels (b) auf dem keratinischen Material, wobei das Mittel (b) einen pH-Wert von 1,5 bis 5,5 besitzt und enthält: Wasser, und
(b1) mindestens eine Säure, und
(b2) gegebenenfalls mindestens ein filmbildendes Polymer,
mit der Maßgabe, dass mindestens eines der Mittel (a) und/oder (b) mindestens ein filmbildendes Polymer enthält,
**dadurch gekennzeichnet, dass** zunächst das Mittel (a) angewendet wird, danach das Mittel (b) angewendet wird, wobei der Zeitraum zwischen der Anwendung der Mittel (a) und (b) bei maximal 24 Stunden liegt.

2. Verfahren nach Anspruch 1, **dadurch gekennzeichnet, dass** das Mittel (a) mindestens ein aminofunktionalisiertes Silikonpolymer (a1) mit mindestens einer sekundären Aminogruppe enthält.

3. Verfahren nach einem der Ansprüche 1 bis 2, **dadurch gekennzeichnet, dass** das Mittel (a) mindestens ein aminofunktionalisiertes Silikonpolymer (a1) enthält, das mindestens eine Struktureinheit der Formel (Si-Amino) umfasst, 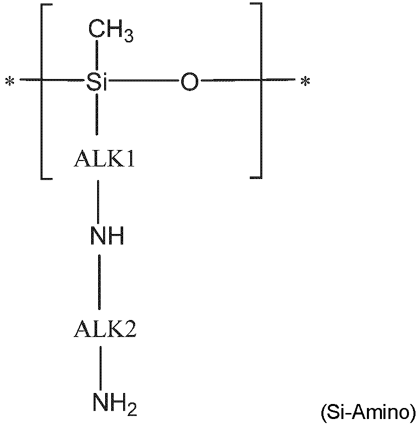 wobei
ALK1 und ALK2 unabhängig voneinander für eine lineare oder verzweigte, zweiwertige C₁-C₂₀-Alkylengruppe stehen.

4. Verfahren nach einem der Ansprüche 1 bis 3, **dadurch gekennzeichnet, dass** das Mittel (a) mindestens ein aminofunktionalisiertes Silikonpolymer (a1) enthält, das Struktureinheiten der Formel (Si-I) und der Formel (Si-II) umfasst

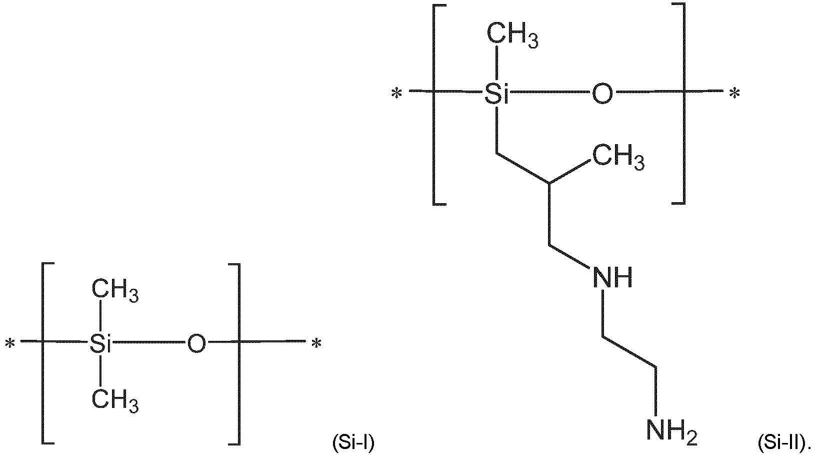

5. Verfahren nach einem der Ansprüche 1 bis 4, **dadurch gekennzeichnet, dass** das Mittel (a) - bezogen auf das Gesamtgewicht des Mittels (a) - ein oder mehrere aminofunktionalisierte Silikonpolymere in einer Gesamtmenge von 0,1 bis 8,0 Gew.-%, bevorzugt 0,02 bis 5,0 Gew.-%, weiter bevorzugt von 0,1 bis 3,0 Gew.-% und ganz besonders bevorzugt von 0,05 bis 3,5 Gew.-% enthält.

6. Verfahren nach einem der Ansprüche 1 bis 5, **dadurch gekennzeichnet, dass** das Mittel (a) mindestens eine farbgebende Verbindung (a2) aus der Gruppe der anorganischen Pigmente enthält, das ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

7. Verfahren nach einem der Ansprüche 1 bis 6, **dadurch gekennzeichnet, dass** das Mittel (a) mindestens eine farbgebende Verbindung (a2) aus der Gruppe der organischen Pigmente enthält, die ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

8. Verfahren nach einem der Ansprüche 1 bis 7, **dadurch gekennzeichnet, dass** das Mittel (a) mindestens ein nichtionisches, filmbildendes, hydrophiles Polymer (a3) enthält.

9. Verfahren nach einem der Ansprüche 1 bis 8, **dadurch gekennzeichnet, dass** das Mittel (a) mindestens ein filmbildendes Polymer (a3) enthält, das ausgewählt ist aus der Gruppe aus Polyvinylpyrrolidon (PVP) und den Copolymeren des Polyvinylpyrrolidons.

10. Verfahren nach einem der Ansprüche 1 bis 9, **dadurch gekennzeichnet, dass** das Mittel (a) mindestens ein filmbildendes Polymer (a3) enthält, das ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/Ethylen-Copolymeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren, explizit ganz besonders bevorzugt Polyvinylpyrrolidon (PVP).

11. Verfahren nach einem der Ansprüche 1 bis 10, **dadurch gekennzeichnet, dass** das Färbemittel (a) mindestens ein Alkalisierungsmittel aus der Gruppe aus Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat enthält.

12. Verfahren nach einem der Ansprüche 1 bis 11, **dadurch gekennzeichnet, dass** das Mittel (a) Wasser enthält und einen pH-Wert von 7,0 bis 11,5 bevorzugt von 8,0 bis 11,0, und besonders bevorzugt von 8,5 bis 10,5 besitzt.

13. Verfahren nach einem der Ansprüche 1 bis 12, **dadurch gekennzeichnet, dass** das Mittel (b) mindestens eine Säure (b1) enthält, die ausgewählt ist aus der Gruppe aus Zitronensäure, Weinsäure, Äpfelsäure, Milchsäure, Essigsäure, Methansulfonsäure, Benzoesäure, Malonsäure, Oxalsäure, 1-Hydroxyethan-1,1-diphosphonsäure, Schwefelsäure, Salzsäure und Phosphorsäure.

14. Verfahren nach einem der Ansprüche 1 bis 13, **dadurch gekennzeichnet, dass** das Mittel (b) Wasser enthält und einen pH-Wert von 2,0 bis 4,8, und bevorzugt von 2,5 bis 4,5 besitzt.

15. Verfahren nach einem der Ansprüche 1 bis 14, **dadurch gekennzeichnet, dass** das Nachbehandlungsmittel (b) mindestens ein filmbildendes Polymer (b2) enthält, das ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/Ethylen-Copolymeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren, der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

16. Verfahren nach einem der Ansprüche 1 bis 15, **dadurch gekennzeichnet, dass** zunächst das Mittel (a) angewendet wird, danach das Mittel (b) angewendet wird, wobei der Zeitraum zwischen der Anwendung der Mittel (a) und (b) bei maximal 12 Stunden und bevorzugt bei maximal 6 Stunden liegt.

17. Verfahren nach einem der Ansprüche 1 bis 16, umfassend die folgenden Schritte in der angegebenen Reihenfolge
(1) Anwendung des Färbemittels (a) auf dem keratinischen Material,
(2) Einwirken lassen des Mittels (a) für einen Zeitraum von 10 Sekunden bis 10 Minuten, bevorzugt von 10 Sekunden bis 5 Minuten,
(3) gegebenenfalls Ausspülen des keratinischen Materials mit Wasser,
(4) Anwendung des Nachbehandlungsmittels (b) auf dem keratinischen Material,
(5) Einwirken lassen des Mittels (b) für einen Zeitraum von 30 Sekunden bis 30 Minuten, bevorzugt von 30 Sekunden bis 10 Minuten, und
(6) Ausspülen des keratinischen Materials mit Wasser.

## Claims

1. A method for dyeing keratinous material, in particular human hair, comprising the following steps:
- applying a coloring agent (a) to the keratinous material, agent (a) containing:
(a1) at least one amino-functionalized silicone polymer, and
(a2) at least one dyeing compound from the group of inorganic and/or organic pigments, and
(a3) optionally at least one film-forming polymer which is different from (a1),
- applying a post-treatment agent (b) to the keratinous material, agent (b) having a pH of 1.5 to 5.5 and containing: water, and
(b1) at least one acid, and
(b2) optionally at least one film-forming polymer,
with the proviso that at least one of the agents (a) and/or (b) contains at least one film-forming polymer,
**characterized in that** first agent (a) is applied, then agent (b) is applied, the period of time between the application of agents (a) and (b) being at most 24 hours.

2. The method according to claim 1, **characterized in that** agent (a) contains at least one amino-functionalized silicone polymer (a1) having at least one secondary amino group.

3. The method according to one of claims 1 to 2, **characterized in that** agent (a) contains at least one amino-functionalized silicone polymer (s1) comprising at least one structural unit of formula (Si-amino), 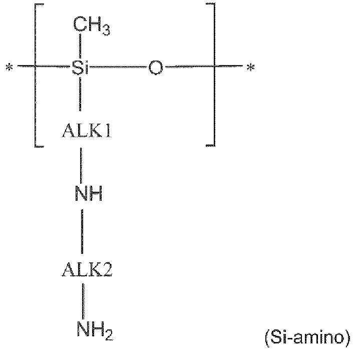 where
ALK1 and ALK2 represent, independently of one another, a linear or branched, bivalent C₁-C₂₀ alkylene group.

4. The method according to one of claims 1 to 3, **characterized in that** agent (a) contains at least one amino-functionalized silicone polymer (a1) comprising structural units of formula (Si-I) and of formula (Si-II)

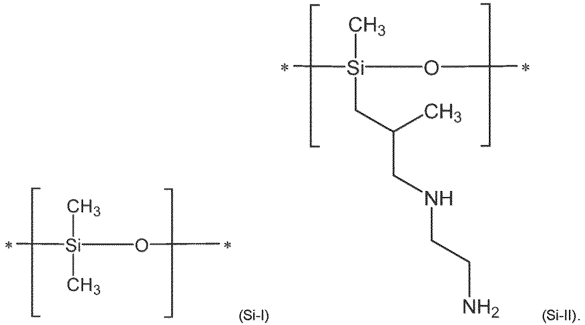

5. The method according to one of claims 1 to 4, **characterized in that** agent (a) contains, based on the total weight of agent (a), one or more amino-functionalized silicone polymers in a total amount of 0.1 to 8.0 wt.%, preferably 0.02 to 5.0 wt.%, more preferably 0.1 to 3.0 wt.%, and very particularly preferably 0.05 to 3.5 wt.%.

6. The method according to one of claims 1 to 5, **characterized in that** agent (a) contains at least one dyeing compound (a2) from the group of inorganic pigments selected from the group of colored metal oxides, metal hydroxides, metal oxide hydrates, silicates, metal sulfides, complex metal cyanides, metal sulphates, bronze pigments and/or from mica-based colored pigments which are coated with at least one metal oxide and/or a metal oxychloride.

7. The method according to one of claims 1 to 6, **characterized in that** agent (a) contains at least one dyeing compound (a2) from the group of organic pigments, selected from the group consisting of carmine, quinacridone, phthalocyanine, sorghum, blue pigments with the Color Index numbers CI 42090, CI 69800, CI 69825, CI 73000, CI 74100 or CI 74160, yellow pigments with the Color Index numbers CI 11680, CI 11710, CI 15985, CI 19140, CI 20040, CI 21100, CI 21108, CI 47000 or CI 47005, green pigments with the Color Index numbers CI 61565, CI 61570 or CI 74260, orange pigments with the Color Index numbers CI 11725, CI 15510, CI 45370 or CI 71105, and red pigments with the Color Index numbers CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 and/or CI 75470.

8. The method according to one of claims 1 to 7, **characterized in that** agent (a) contains at least one non-ionic, film-forming, hydrophilic polymer (a3).

9. The method according to one of claims 1 to 8, **characterized in that** agent (a) contains at least one film-forming polymer (a3) selected from the group consisting of polyvinylpyrrolidone (PVP) and the copolymers of the polyvinylpyrrolidone.

10. The method according to one of claims 1 to 9, **characterized in that** agent (a) contains at least one film-forming polymer (a3) selected from the group consisting of polyvinylpyrrolidone (PVP), vinylpyrrolidone/vinyl acetate copolymers, vinylpyrrolidone/styrene copolymers, vinylpyrrolidone/ethylene copolymers, vinylpyrrolidone/propylene copolymers, vinylpyrrolidone/vinylcaprolactam copolymers, vinylpyrrolidone/vinylformamide copolymers and/or vinylpyrrolidone/vinyl alcohol copolymers, explicitly and particularly preferably polyvinylpyrrolidone (PVP).

11. The method according to one of claims 1 to 10, **characterized in that** the coloring agent (a) contains at least one alkalizing agent from the group consisting of ammonia, 2-aminoethan-1-ol (monoethanolamine), 3-aminopropan-1-ol, 4-aminobutan-1-ol, 5-aminopentan-1-ol, 1-aminopropan-2-ol, 1-aminobutan-2-ol, 1-aminopentan-2-ol, 1-aminopentan-3-ol, 1-aminopentan-4-ol, 3-amino-2-methylpropan-1-ol, 1-amino-2-methylpropan-2-ol, 3-aminopropane-1,2-diol, 2-amino-2-methylpropane-1,3-diol, arginine, lysine, ornithine, histidine, sodium hydroxide, potassium hydroxide, calcium hydroxide, barium hydroxide, sodium phosphate, potassium phosphate, sodium silicate, sodium metasilicate, potassium silicate, sodium carbonate and potassium carbonate.

12. The method according to one of claims 1 to 11, **characterized in that** agent (a) contains water and has a pH in the range of 7.0 to 11.5, preferably of 8.0 to 11.0, and particularly preferably of 8.5 to 10.5.

13. The method according to one of claims 1 to 12, **characterized in that** agent (b) contains at least one acid (b1) selected from the group consisting of citric acid, tartaric acid, malic acid, lactic acid, acetic acid, methanesulfonic acid, benzoic acid, malonic acid, oxalic acid, 1-hydroxyethane-1,1-diphosphonic acid, sulfuric acid, hydrochloric acid and phosphoric acid.

14. The method according to one of claims 1 or 13, **characterized in that** agent (b) contains water and has a pH in the range of 2.0 to 4.8, preferably of 2.5 to 4.5.

15. The method according to one of claims 1 to 14, **characterized in that** the after-treatment agent (b) contains at least one film-forming polymer (b2) which is selected from the group consisting of polyvinylpyrrolidone (PVP), vinylpyrrolidone/vinyl acetate copolymers, vinylpyrrolidone/styrene copolymers, vinylpyrrolidone/ethylene copolymers, vinylpyrrolidone/propylene copolymers, vinylpyrrolidone/vinylcaprolactam copolymers, vinylpyrrolidone/vinylformamide copolymers and/or vinylpyrrolidone/vinyl alcohol copolymers, copolymers of acrylic acid, copolymers of methacrylic acid, homopolymers or copolymers of acrylic acid esters, homopolymers or copolymers of methacrylic acid esters, homopolymers or copolymers of acrylic acid amides, homopolymers or copolymers of methacrylic acid amides, copolymers of vinylpyrrolidone, copolymers of vinyl alcohol, copolymers of vinyl acetate, homopolymers or copolymers of ethylene, homopolymers or copolymers of propylene, homopolymers or copolymers of styrene, polyurethanes, polyesters and/or polyamides.

16. The method according to one of claims 1 to 15, **characterized in that** first agent (a) is applied and then agent (b) is applied, the period of time between the application of agents (a) and (b) being at most 12 hours, and preferably at most 6 hours.

17. The method according to one of claims 1 to 16, comprising the following steps in the stated sequence:
(1) applying the coloring agent (a) to the keratinous material,
(2) allowing agent (a) to act for a period of 10 seconds to 10 minutes, preferably 10 seconds to 5 minutes,
(3) optionally rinsing the keratinous material with water,
(4) applying the after-treatment agent (b) to the keratinous material,
(5) allowing agent (b) to act for a period of 30 seconds to 30 minutes, preferably 30 seconds to 10 minutes, and
(6) rinsing the keratinous material with water.

## Revendications

1. Procédé permettant de colorer de la matière kératinique, en particulier des cheveux humains, comprenant les étapes suivantes :
- application d'un agent colorant (a) sur la matière kératinique, dans lequel l'agent (a) contient :
(a1) au moins un polymère de silicone aminofonctionnalisé, et
(a2) au moins un composé colorant du groupe des pigments inorganiques et/ou organiques, et
(a3) éventuellement au moins un polymère filmogène qui est différent de (a1),
- application d'un agent de post-traitement (b) sur la matière kératinique, dans lequel l'agent (b) possède un pH allant de 1,5 à 5,5 et contient : de l'eau, et
(b1) au moins un acide, et
(b2) éventuellement au moins un polymère filmogène,
à condition qu'au moins l'un parmi les agents (a) et/ou (b) contienne au moins un polymère filmogène,
**caractérisé en ce que** l'agent (a) est d'abord appliqué, puis l'agent (b) est appliqué, dans lequel la durée entre l'application des agents (a) et (b) est au maximum de 24 heures.

2. Procédé selon la revendication 1, **caractérisé en ce que** l'agent (a) contient au moins un polymère de silicone aminofonctionnalisé (a1) comportant au moins un groupe amine secondaire.

3. Procédé selon l'une des revendications 1 à 2, **caractérisé en ce que** l'agent (a) contient au moins un polymère de silicone aminofonctionnalisé (a1) qui comprend au moins un motif structural de formule (Si-Amino), 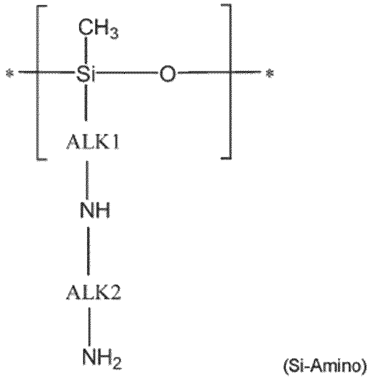 où
ALK1 et ALK2 représentent, indépendamment l'un de l'autre, un groupe alkylène en C₁-C₂₀ divalent, linéaire ou ramifié.

4. Procédé selon l'une des revendications 1 à 3, **caractérisé en ce que** l'agent (a) contient au moins un polymère de silicone aminofonctionnalisé (a1) qui comprend des motifs structuraux de formule (Si-I) et de formule (Si-II)

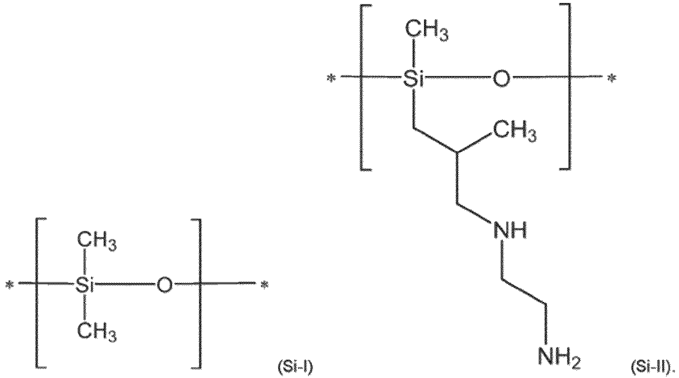

5. Procédé selon l'une des revendications 1 à 4, **caractérisé en ce que** l'agent (a) contient, par rapport au poids total de l'agent (a), un ou plusieurs polymères de silicone aminofonctionnalisés en une quantité totale allant de 0,1 à 8,0 % en poids, de préférence de 0,02 à 5,0 % en poids, plus préférablement de 0,1 à 3,0 % en poids et de manière tout particulièrement préférée de 0,05 à 3,5 % en poids.

6. Procédé selon l'une des revendications 1 à 5, **caractérisé en ce que** l'agent (a) contient au moins un composé colorant (a2) du groupe des pigments inorganiques qui est choisi dans le groupe constitué d'oxydes métalliques colorés, hydroxydes métalliques, oxydes métalliques hydratés, silicates, sulfures métalliques, cyanures métalliques complexes, sulfates métalliques, pigments de bronze et/ou de pigments colorés à base de mica recouverts d'au moins un oxyde métallique et/ou un oxychlorure métallique.

7. Procédé selon l'une des revendications 1 à 6, **caractérisé en ce que** l'agent (a) contient au moins un composé colorant (a2) du groupe des pigments organiques qui est choisi dans le groupe constitué de carmin, quinacridone, phtalocyanine, sorgho, pigments bleus comportant les numéros d'indice de couleur CI 42090, CI 69800, CI 69825, CI 73000, CI 74100, CI 74160, pigments jaunes comportant les numéros d'indice de couleur CI 11680, CI 11710, CI 15985, CI 19140, CI 20040, CI 21100, CI 21108, CI 47000, CI 47005, pigments verts comportant les numéros d'indice de couleur CI 61565, CI 61570, CI 74260, pigments orange comportant les numéros d'indice de couleur CI 11725, CI 15510, CI 45370, CI 71105, pigments rouges comportant les numéros d'indice de couleur CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

8. Procédé selon l'une des revendications 1 à 7, **caractérisé en ce que** l'agent (a) contient au moins un polymère hydrophile, filmogène et non ionique (a3).

9. Procédé selon l'une des revendications 1 à 8, **caractérisé en ce que** l'agent (a) contient au moins un polymère filmogène (a3) qui est choisi dans le groupe constitué de polyvinylpyrrolidone (PVP) et des copolymères de la polyvinylpyrrolidone.

10. Procédé selon l'une des revendications 1 à 9, **caractérisé en ce que** l'agent (a) contient au moins un polymère filmogène (a3) qui est choisi dans le groupe constitué de polyvinylpyrrolidone (PVP), copolymères de vinylpyrrolidone/acétate de vinyle, copolymères de vinylpyrrolidone/styrène, copolymères de vinylpyrrolidone/éthylène, copolymères de vinylpyrrolidone/propylène, copolymères de vinylpyrrolidone/vinylcaprolactame, copolymères de vinylpyrrolidone/vinylformamide et/ou copolymères de vinylpyrrolidone/alcool vinylique, de manière explicite et tout particulièrement préférée est la polyvinylpyrrolidone (PVP).

11. Procédé selon l'une des revendications 1 à 10, **caractérisé en ce que** l'agent colorant (a) contient au moins un agent alcalinisant du groupe constitué d'ammoniac, 2-aminoéthan-1-ol (monoéthanolamine), 3-aminopropan-1-ol, 4-aminobutan-1-ol, 5-aminopentan-1-ol, 1-aminopropan-2-ol, 1-aminobutan-2-ol, 1-aminopentan-2-ol, 1-aminopentan-3-ol, 1-aminopentan-4-ol, 3-amino-2-méthylpropan-1-ol, 1-amino-2-méthylpropan-2-ol, 3-aminopropane-1,2-diol, 2-amino-2-méthylpropane-1,3-diol, arginine, lysine, ornithine, histidine, hydroxyde de sodium, hydroxyde de potassium, hydroxyde de calcium, hydroxyde de baryum, phosphate de sodium, phosphate de potassium, silicate de sodium, métasilicate de sodium, silicate de potassium, carbonate de sodium et carbonate de potassium.

12. Procédé selon l'une des revendications 1 à 11, **caractérisé en ce que** l'agent (a) contient de l'eau et possède un pH allant de 7,0 à 11,5, de préférence de 8,0 à 11,0 et de manière particulièrement préférée de 8,5 à 10,5.

13. Procédé selon l'une des revendications 1 à 12, **caractérisé en ce que** l'agent (b) contient au moins un acide (b1) qui est choisi dans le groupe constitué d'acide citrique, acide tartrique, acide malique, acide lactique, acide acétique, acide méthanesulfonique, acide benzoïque, acide malonique, acide oxalique, acide 1-hydroxyéthane-1,1-diphosphonique, acide sulfurique, acide chlorhydrique et acide phosphorique.

14. Procédé selon l'une des revendications 1 à 13, **caractérisé en ce que** l'agent (b) contient de l'eau et possède un pH allant de 2,0 à 4,8, et de préférence de 2,5 à 4,5.

15. Procédé selon l'une des revendications 1 à 14, **caractérisé en ce que** l'agent de post-traitement (b) contient au moins un polymère filmogène (b2) qui est choisi dans le groupe constitué de polyvinylpyrrolidone (PVP), copolymères de vinylpyrrolidone/acétate de vinyle, copolymères de vinylpyrrolidone/styrène, copolymères de vinylpyrrolidone/éthylène, copolymères de vinylpyrrolidone/propylène, copolymères de vinylpyrrolidone/vinylcaprolactame, copolymères de vinylpyrrolidone/vinylformamide et/ou copolymères de vinylpyrrolidone/alcool vinylique, des copolymères d'acide acrylique, des copolymères d'acide méthacrylique, des homopolymères ou copolymères d'esters d'acide acrylique, des homopolymères ou copolymères d'esters d'acide méthacrylique, des homopolymères ou copolymères d'amides d'acide acrylique, des homopolymères ou copolymères d'amides d'acide méthacrylique, des copolymères de la vinylpyrrolidone, des copolymères de l'alcool vinylique, des copolymères de l'acétate de vinyle, des homopolymères ou copolymères de l'éthylène, des homopolymères ou copolymères du propylène, des homopolymères ou copolymères du styrène, des polyuréthanes, des polyesters et/ou des polyamides.

16. Procédé selon l'une des revendications 1 à 15, **caractérisé en ce que** l'agent (a) est d'abord appliqué, puis l'agent (b) est appliqué, dans lequel la durée entre l'application des agents (a) et (b) est au maximum de 12 heures et de préférence au maximum de 6 heures.

17. Procédé selon l'une des revendications 1 à 16, comprenant les étapes suivantes dans l'ordre indiqué
(1) application de l'agent colorant (a) sur la matière kératinique,
(2) attente de l'action de l'agent (a) pendant une durée allant de 10 secondes à 10 minutes, de préférence de 10 secondes à 5 minutes,
(3) éventuellement, rinçage de la matière kératinique avec de l'eau,
(4) application de l'agent de post-traitement (b) sur la matière kératinique,
(5) attente de l'action de l'agent (b) pendant une durée allant de 30 secondes à 30 minutes, de préférence de 30 secondes à 10 minutes, et
(6) rinçage de la matière kératinique avec de l'eau.
